# EUROPEAN PATENT SPECIFICATION

(11) **EP 1 224 179 B1**
(45) Date of publication and mention of the grant of the patent: **14.07.2004**
(21) Application number: 00962776.1
(22) Date of filing: 09.10.2000
(51) Int. Cl.: C07D 401/04, A61K 31/4709, A61P 9/10

(54) **SODIUM-HYDROGEN EXCHANGER TYPE 1 INHIBITOR CRYSTALS**
TYPE 1 INHIBITORKRISTALLE ALS INHIBITOREN DES NATRIUM-PROTONEN AUSTAUSCHS
CRISTAUX D'INHIBITEURS DE L'ECHANGEUR SODIUM-HYDROGENE DE TYPE 1

(30) Priority: 29.10.1999 US 162374 P
(43) Date of publication of application: 24.07.2002
(73) Proprietor: Pfizer Products Inc., Groton, Connecticut 06340 (US)
(72) Inventor: BROSTROM, Lyle, Robinson, Lincolnshire, IL 60069 (US); CONNOLLY, Terrence, Joseph, Eastern Point Road, Groton, CT 06340 (US); LI, Zheng, Jane, Pfizer Global Research and Dev., Groton, CT 06340 (US); ORRILL, Susan, L., Pfizer Global Research and Dev., Groton, CT 06340 (US); SHAH, Bharat, K., Pfizer Global Research and Dev., Groton, CT 06340 (US)
(74) Representative: Sewell, Richard Charles
(86) International application number: PCT/IB2000/001460
(87) International publication number: WO 2001/030759

(56) References cited:
- WO-A-99/43663

## Description

### BACKGROUND OF INVENTION

This invention relates to sodium-hydrogen exchanger type 1 (NHE-1) inhibitors and particularly, crystals of such inhibitors.

Myocardial ischemic injury can occur in out-patient as well as in perioperative settings and can lead to the development of sudden death, myocardial infarction or congestive heart failure. There is an unmet medical need to prevent or minimize myocardial ischemic injury, particularly perioperative myocardial infarction. Such a therapy is anticipated to be life-saving and reduce hospitalizations, enhance quality of life and reduce overall health care costs of high risk patients.

Pharmacological cardioprotection would reduce the incidence and progression of myocardial infarction and dysfunction occurring in these surgical settings (perioperatively). In addition to reducing myocardial damage and improving post-ischemic myocardial function in patients with ischemic heart disease, cardioprotection would also decrease the incidence of cardiac morbidity and mortality due to myocardial infarction and dysfunction in patients "at risk" (such as greater than 65 years, exercise intolerant, coronary artery disease, diabetes mellitus, hypertension) that require non-cardiac surgery.

The mechanism(s) responsible for the myocardial injury observed after ischemia and reperfusion is not fully understood.

A variety of publications have disclosed the use of guanidine derivatives as useful for the treatment of, for example, arrhythmias.

A recent published patent application, PCT/IB99/00206 published as WO 99/43663 on September 2, 1999, the disclosure of which is hereby incorporated by reference, discloses a variety of NHE-1 inhibitors including [5-cyclopropyl-1-(quinolin-5-yl)-1H-pyrazole-4-carbonyl]guanidine. The publication further states that "preferred salts of the immediately preceding compound are the mono- or di-mesylate salts."

PCT/JP97/04650 application published on June 25, 1998 discloses N-[(substituted five-membered heteroaryl)]guanidine compounds which are disclosed to be useful as inhibitors of Na⁺/H⁺ exchange and consequently effective for the treatment of various diseases such as hypertension, arrhythmia, angina pectoris, myocardial infarct, arteriosclerosis, and complications of diabetes.

Thus, there is clearly a need and a continuing search in this field of art for compounds for the treatment of perioperative myocardial ischemia and, accordingly, new crystal forms of such compounds.

### SUMMARY OF THE INVENTION

This invention is directed to a crystal of Formula I

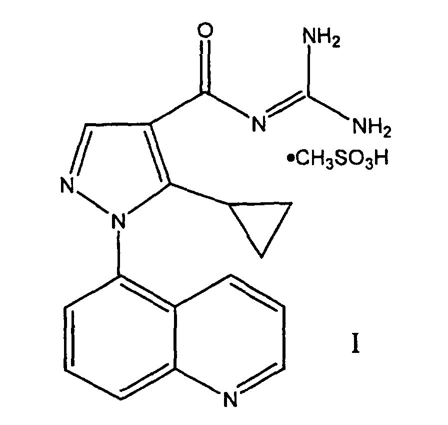

Alternatively, the above salt is named as N-(5-cyclopropyl-1-quinolin-5-yl-1H-pyrazole-4-carbonyl)-guanidine, monomesylate salt.

Another aspect of this invention is directed to an anhydrous crystal form of the Formula I salt.

Another aspect of this invention is directed to an anhydrous crystal form A of the Formula I salt having the X-ray diffraction d-spacing of Table III below.

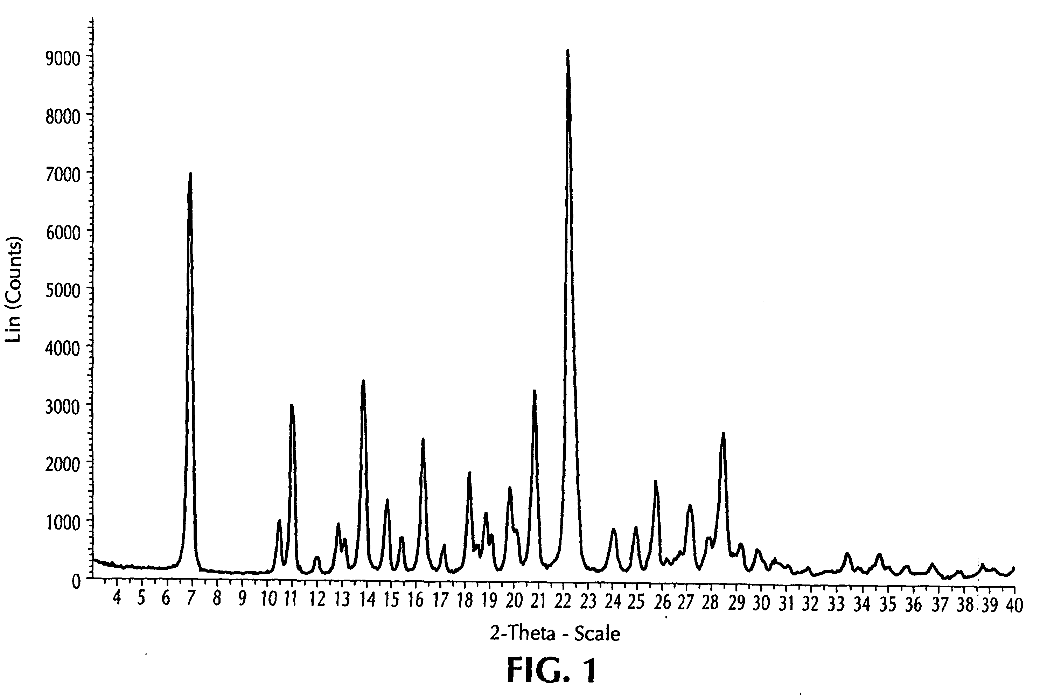

Another aspect of this invention is directed to an anhydrous crystal form A of the Formula I salt having the X-ray powder diffraction pattern as shown in Figure 1.

Another aspect of this invention is directed to an anhydrous crystal form D of the Formula I salt having the X-ray diffraction d-spacing of Table II below.

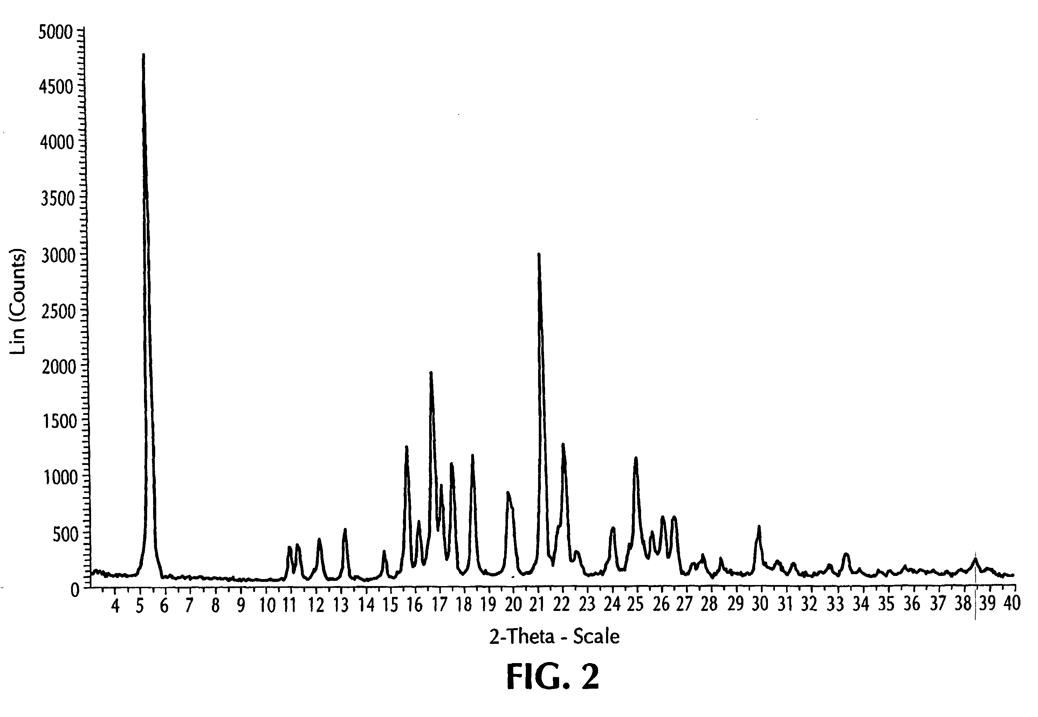

Another aspect of this invention is directed to an anhydrous crystal form D of the Formula I salt having the X-ray powder diffraction pattern as shown in Figure 2.

Another aspect of this invention is directed to a hemihydrate crystal form of the Formula I salt preferably having the X-ray diffraction d-spacing of Table IV below.

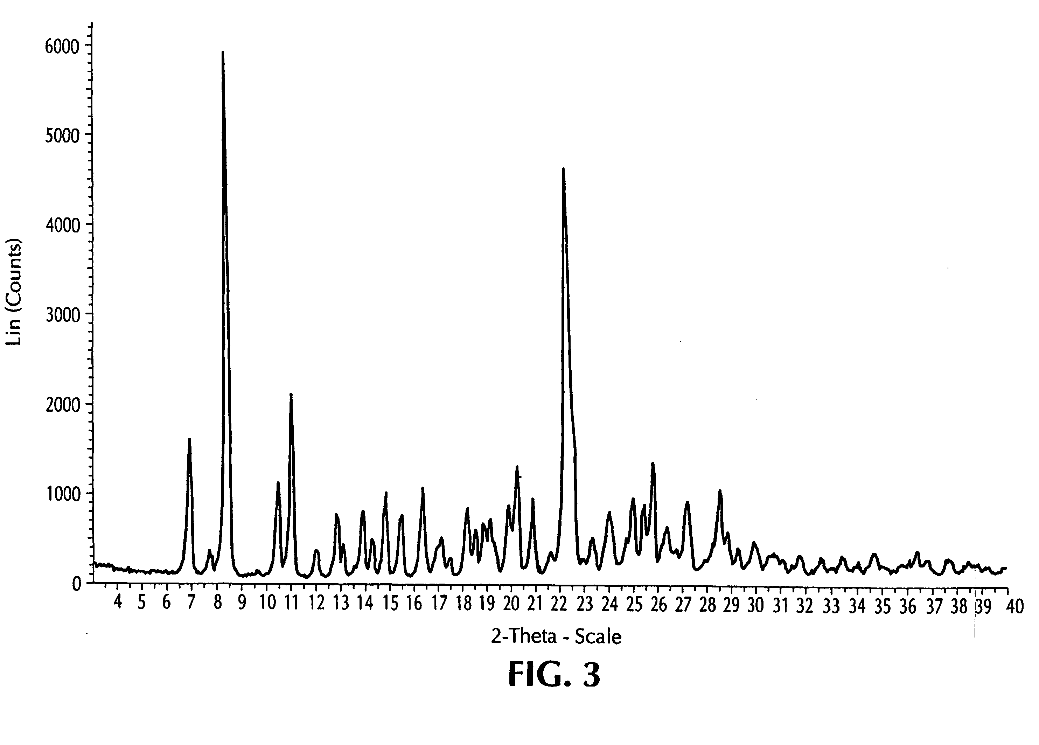

Another aspect of this invention is directed to a hemihydrate crystal form of the Formula I salt having the X-ray powder diffraction pattern as shown in Figure 3.

In the text herein, including the following uses, pharmaceutical compositions, combinations and kits, reference is made to a crystal of Formula I. While it is understood that if the crystal is in solution, the crystal form is not present (in contrast to e.g., a dry tablet formulation), the following uses, pharmaceutical compositions, combinations and kits are intended to include a use or formulation resulting from such crystal (e.g., an I.V. solution made from the crystal).

Another aspect of this invention is the use of a crystal of Formula I for the preparation of a medicament for treating a mammal (e.g., human) having a disease or condition mediated by NHE-1.

Another aspect of this invention is directed to the use of a crystal of Formula I for the preparation of a medicament for reducing tissue damage (e.g., substantially preventing tissue damage, inducing tissue protection) resulting from ischemia in a mammal (e.g., a female or male human).

Preferred ischemic tissues taken individually or as a group are wherein the ischemic tissue is cardiac, brain, liver, kidney, lung, gut, skeletal muscle, spleen, pancreas, nerve, spinal cord, retina tissue, the vasculature, or intestinal tissue.

An especially preferred ischemic tissue is cardiac tissue.

It is especially preferred that the medicament is to be administered to prevent perioperative myocardial ischemic injury.

Preferably, the medicament is to be administered prophylactically.

The ischemic damage may occur during organ transplantation either to the organ or the patient.

Preferably, the medicament is to be administered prior to, during and/or shortly after, cardiac surgery or non-cardiac surgery.

In one aspect of this invention the medicament is to be administered locally.

A preferred dosage is about 0.001 to 100 mg/kg/day of a Formula I crystal. An especially preferred dosage is about 0.01 to 50 mg/kg/day of a Formula I crystals.

Another aspect of this invention is directed to the use of a crystal of Formula I for the preparation of a medicament for reducing myocardial tissue damage (e.g., substantially preventing tissue damage, inducing tissue protection) during surgery (e.g., coronary artery bypass grafting (CABG) surgeries, vascular surgeries, percutaneous transluminal coronary angioplasty (PTCA), organ transplantation, or other non-cardiac surgeries) in a mammal (e.g., a female or male human).

Another aspect of this invention is directed to the use of a crystal of Formula I for the preparation of a medicament for reducing myocardial tissue damage (e.g., substantially preventing tissue damage, inducing tissue protection) in patients presenting with ongoing cardiac (acute coronary syndromes, e.g. myocardial infarction or unstable angina) or cerebral ischemic events (e.g., stroke).

Another aspect of this invention is directed to the use of a crystal of Formula I for the preparation of a medicament for reducing myocardial tissue damage (e.g., substantially preventing tissue damage, inducing tissue protection) on a chronic basis in a patient with diagnosed coronary heart disease (e.g., previous myocardial infarction or unstable angina) or patients who are at high risk for myocardial infarction (e.g., age > 65 and two or more risk factors for coronary heart disease).

Another aspect of this invention is directed to the use of a crystal of Formula I for the preparation of a medicament for preventing ischemic damage by chronic oral administration to a mammal in need of such treatment.

Another aspect of this invention is directed to the use of a crystal of Formula I for the preparation of a medicament for treating cardiovascular diseases in a mammal (e.g., a female or male human).

Another aspect of this invention is directed to the use of a crystal of Formula I for the preparation of a medicament for treating arteriosclerosis in a mammal (e.g., a female or male human).

Another aspect of this invention is directed to the use of a crystal of Formula I for the preparation of a medicament for treating hypertension in a mammal (e.g., a female or male human).

Another aspect of this invention is directed to the use of a crystal of Formula I for the preparation of a medicament for treating arrhythmia in a mammal (e.g., a female or male human).

Another aspect of this invention is directed to the use of a crystal of Formula I for the preparation of a medicament for treating angina pectoris in a mammal (e.g., a female or male human).

Another aspect of this invention is directed to the use of a crystal of Formula I for the preparation of a medicament for treating cardiac hypertrophy in a mammal (e.g., a female or male human).

Another aspect of this invention is directed to the use of a crystal of Formula I for the preparation of a medicament for treating renal diseases in a mammal (e.g., a female or male human).

Another aspect of this invention is directed to the use of a crystal of Formula I for the preparation of a medicament for treating diabetic complications in a mammal (e.g., a female or male human).

Another aspect of this invention is directed to the use of a crystal of Formula I for the preparation of a medicament for treating restenosis in a mammal (e.g., a female or male human).

Another aspect of this invention is directed to the use of a crystal of Formula I for the preparation of a medicament for treating diseases of cell proliferation in a mammal (e.g., a female or male human).

Another aspect of this invention is directed to the use of a crystal of Formula I for the preparation of a medicament for treating cancerous diseases in a mammal (e.g., a female or male human).

Another aspect of this invention is directed to the use of a crystal of Formula I for the preparation of a medicament for treating fibrotic diseases in a mammal (e.g., a female or male human).

Another aspect of this invention is directed to the use of a crystal of Formula I for the preparation of a medicament for treating glomerular nephrosclerosis in a mammal (e.g., a female or male human).

Another aspect of this invention is directed to the use of a crystal of Formula I for the preparation of a medicament for treating organ hypertrophies or hyperplasias in a mammal (e.g., a female or male human).

Another aspect of this invention is directed to the use of a crystal of Formula I for the preparation of a medicament for treating pulmonary fibrosis in a mammal (e.g., a female or male human).

Another aspect of this invention is directed to the use of a crystal of Formula I for the preparation of a medicament for treating cerebro ischemic disorders in a mammal (e.g., a female or male human).

Another aspect of this invention is directed to the use of a crystal of Formula I for the preparation of a medicament for treating myocardial stunning in a mammal (e.g., a female or male human).

Another aspect of this invention is directed to the use of a crystal of Formula I for the preparation of a medicament for treating myocardial dysfunction in a mammal (e.g., a female or male human).

Another aspect of this invention is directed to the use of a crystal of Formula I for the preparation of a medicament for treating cerebrovascular diseases in a mammal (e.g., a female or male human).

This invention is also directed to pharmaceutical compositions which comprise a therapeutically effective amount of a crystal of Formula I and a pharmaceutically acceptable carrier, vehicle or diluent.

This invention is also directed to pharmaceutical compositions for the reduction of tissue damage resulting from ischemia which comprise a therapeutically effective amount of a crystal of Formula I and a pharmaceutically acceptable carrier, vehicle or diluent.

Yet another aspect of this invention are combinations of a crystal of Formula I and other compounds as described below.

Yet another aspect of this invention is directed to therapeutically effective amounts of a pharmaceutical composition comprising a crystal of Formula I and a cardiovascular agent and particularly to such a composition for the reduction of tissue damage resulting from tissue ischemia in mammals (e.g., humans, male or female).

Another aspect of this invention is the use of
a. a crystal of Formula I; and
b. a cardiovascular agent
for the preparation of a medicament for reducing tissue damage (e.g., substantially preventing tissue damage, inducing tissue protection) resulting from or which could result from ischemia in a mammal (e.g., a female or male human) wherein the amounts of the first and second compounds result in a therapeutic effect.

Another aspect of this invention is a kit comprising:
a. a crystal of Formula I and a pharmaceutically acceptable carrier, vehicle or diluent in a first unit dosage form;
b. a cardiovascular agent and a pharmaceutically acceptable carrier, vehicle or diluent in a second unit dosage form; and
c. means for containing said first and second dosage forms wherein the amounts of the first and second compounds result in a therapeutic effect.

In the above combination compositions, combination methods and kits, preferably the cardiovascular agents are, for example, β-blockers (e.g., acebutolol, atenolol, bopindolol, labetolol, mepindolol, nadolol, oxprenol, pindolol, propranolol, sotalol), calcium channel blockers (e.g., amlodipine, nifedipine, nisoldipine, nitrendipine, verapamil), potassium channel openers, adenosine, adenosine agonists, ACE inhibitors (e.g., captopril, enalapril), nitrates (e.g., isosorbide dinitrate, isosorbide 5-mononitrate, glyceryl trinitrate), diuretics (e.g., hydrochlorothiazide, indapamide, piretanide, xipamide), glycosides (e.g., digoxin, metildigoxin), thrombolytics (e.g., tPA), platelet inhibitors (e.g., reopro), aspirin, dipyridamol, potassium chloride, clonidine, prazosin or adenosine A₃ receptor agonists.

This invention is also directed to a pharmaceutical combination composition comprising a therapeutically effective amount of a composition comprising:
a first compound, said first compound being a crystal of Formula I;
a second compound, said second compound being a glycogen phosphorylase inhibitor; and/or optionally
a pharmaceutical carrier, vehicle or diluent.

Another aspect of this invention is the use of
a. a first compound, said first compound being a crystal of Formula I; and
b. a second compound, said second compound being a glycogen-phosphorylase inhibitor,
for the preparation of a medicament for reducing tissue damage (e.g., substantially preventing tissue damage, inducing tissue protection) resulting from or which could result from ischemia in a mammal (e.g., a female or male human) wherein the amounts of the first and second compounds result in a therapeutic effect.

Another aspect of this invention is a kit comprising:
a. a crystal of Formula I and a pharmaceutically acceptable carrier, vehicle or diluent in a first unit dosage form;
b. a glycogen phosphorylase inhibitor and a pharmaceutically acceptable carrier, vehicle or diluent in a second unit dosage form; and
c. means for containing said first and second dosage forms wherein the amounts of the first and second compounds result in a therapeutic effect.

In the above combination compositions, combination methods and kits preferred glycogen phosphorylase inhibitors are
5-chloro-1H-indole-2-carboxylic acid [(1S)-((R)-hydroxy-dimethylcarbamoyl-methyl)-2-phenyl-ethyl]-amide,
5,6-dichloro-1H-indole-2-carboxylic acid {(1S)-[(R)-hydroxy-(methoxy-methyl-carbamoyl)-methyl]-2-phenyl-ethyl}-amide,
5-chloro-1H-indole-2-carboxylic acid {(1S)-[(R)-hydroxy-(methoxy-methyl-carbamoyl)-methyl]-2-phenyl-ethyl}-amide,
5-chloro-1H-indole-2-carboxylic acid ((1S)-{(R)-hydroxy-[(2-hydroxy-ethyl)-methyl-carbamoyl]-methyl}-2-phenyl-ethyl)-amide,
5-chloro-1H-indole-2-carboxylic acid {(1S)-[(R)-hydroxy-(methyl-pyridin-2-yl-carbamoyl)-methyl]-2-phenyl-ethyl}-amide
5-chloro-1 H-indole-2-carboxylic acid ((1S)-{(R)-hydroxy-[methyl-(2-pyridin-2-yl-ethyl)-carbamoyl]-methyl}-2-phenyl-ethyl)-amide,
5-chloro-1H-indole-2-carboxylic acid [(1S)-benzyl-(2R)-hydroxy-3-(4-methyl-piperazin-1-yl)-3-oxo-propyl]-amide hydrochloride,
5-chloro-1H-indole-2-carboxylic acid [(1S)-benzyl-(2R)-hydroxy-3-(3-hydroxy-azetidin-1-yl)-3-oxo-propyl]-amide,
5-chloro-1 H-indole-2-carboxylic acid ((1S)-benzyl-(2R)-hydroxy-3-isoxazolidin-2-yl-3-oxo-propyl)-amide,
5-chloro-1H-indole-2-carboxylic acid ((1S)-benzyl-(2R)-hydroxy-3-[1,2]oxazinan-2-yl-3-oxo-propyl)-amide,
5-chloro-1H-indole-2-carboxylic acid [(1S)-benzyl-(2R)-hydroxy-3-((3S)-hydroxy-pyrrolidin-1-yl)-3-oxo-propyl]-amide,
5-chloro-1H-indole-2-carboxylic acid [(1S)-benzyl-3-((3S,4S)-dihydroxy-pyrrolidin-1-yl)-(2R)-hydroxy-3-oxo-propyl]-amide,
5-chloro-1H-indole-2-carboxylic acid [(1S)-benzyl-3-((3R,4S)-dihydroxy-pyrrolidin-1-yl)-(2R)-hydroxy-3-oxo-propyl]-amide,
5-chloro-1H-indole-2-carboxylic acid ((1S)-benzyl-(2R)-hydroxy-3-morpholin-4-yl-3-oxo-propyl)-amide,
5-chloro-1H-indole-2-carboxylic acid [(1S)-benzyl-2-(3-hydroxyimino-pyrrolidin-1-yl)-2-oxo-ethyl]-amide,
5-chloro-1H-indole-2-carboxylic acid [2-(cis-3,4-dihydroxy-pyrrolidin-1-yl)-2-oxo-ethyl]-amide,
5-chloro-1H-indole-2-carboxylic acid [2-((3S,4S)-dihydroxy-pyrrolidin-1-yl)-2-oxo-ethyl]-amide,
5-chloro-1H-indole-2-carboxylic acid [(1S)-benzyl-2-(cis-3,4-dihydroxy-pyrrolidin-1-yl)-2-oxo-ethyl]-amide,
5-chloro-1H-indole-2-carboxylic acid [2-(1,1-dioxo-thiazolidin-3-yl)-2-oxo-ethyl]-amide,
5-chloro-1H-indole-2-carboxylic acid (2-oxo-2-thiazolidin-3-yl-ethyl)-amide,
5-chloro-1H-indole-2-carboxylic acid [(1S)-(4-fluoro-benzyl)-2-(4-hydroxy-piperidin-1-yl)-2-oxo-ethyl]-amide,
5-chloro-1H-indole-2-carboxylic acid [(1S)-benzyl-2-((3RS)-hydroxy-piperidin-1-yl)-2-oxo-ethyl]-amide,
5-chlora-1H-indole-2-carboxylic acid [2-oxo-2-((1RS)-oxo-1-thiazolidin-3-yl)-ethyl]-amide,
5-chloro-1H-indole-2-carboxylic acid [(1S)-(2-fluoro-benzyl)-2-(4-hydroxy-piperidin-1-yl)-2-oxo-ethyl]-amide,
5-chloro-1H-indole-2-carboxylic acid [(1S)-benzyl-2-((3S,4S)-dihydroxy-pyrrolidin-1-yl)-2-oxo-ethyl]-amide,
5-chloro-1H-indole-2-carboxylic acid [(1S)-benzyl-2-(3-hydroxy-azetidin-1-yl)-2-oxo-ethyl]-amide,
5-chloro-1H-indole-2-carboxylic acid [(1S)-benzyl-2-(3-hydroxyimino-azetidin-1-yl)-2-oxo-ethyl]-amide and
5-chloro-1H-indole-2-carboxylic acid [(1S)-benzyl-2-(4-hydroxyimino-piperidin-1-yl)-2-oxo-ethyl]-amide.

This invention is also directed to a pharmaceutical combination composition comprising a therapeutically effective amount of a composition comprising:
a first compound, said first compound being a crystal of Formula I;
a second compound, said second compound being an aldose reductase inhibitor; and/or optionally
a pharmaceutical carrier, vehicle or diluent.

Another aspect of this invention is the use of
a. a first compound, said first compound being a crystal of Formula I; and
b. a second compound, said second compound being an aldose reductase inhibitor,
for the preparation of a medicament for reducing tissue damage (e.g., substantially preventing tissue damage, inducing tissue protection) resulting from or which could result from ischemia in a mammal (e.g., a female or male human) wherein the amounts of the first and second compounds result in a therapeutic effect.

Another aspect of this invention is a kit comprising:
a. a crystal of Formula I and a pharmaceutically acceptable carrier, vehicle or diluent in a first unit dosage form;
b. an aldose reductase inhibitor and a pharmaceutically acceptable carrier, vehicle or diluent in a second unit dosage form; and
c. means for containing said first and second dosage forms wherein the amounts of the first and second compounds result in a therapeutic effect.

In the above combination compositions, combination methods and kits a preferred aldose reductase inhibitor is zopolrestat: 1-phthalazineacetic acid, 3,4-dihydro-4-oxo-3-[[5-trifluoromethyl)-2-benzothiazolyl]methyl]-.

This invention is also directed to pharmaceutical compositions which comprise a therapeutically effective amount of a crystal of Formula I and a pharmaceutically acceptable carrier, diluent or excipient.

This invention is also directed to pharmaceutical compositions for the reduction of tissue damage resulting from ischemia which comprise a therapeutically effective amount of a crystal of Formula I and a pharmaceutically acceptable carrier, diluent or excipient.

The term "reduction" is intended to include partial prevention or prevention which, although greater than that which would result from taking no compound or from taking a placebo, is less than 100% in addition to substantially total prevention.

The term "damage resulting from ischemia" as employed herein refers to conditions directly associated with reduced blood flow to tissue, for example due to a clot or obstruction of blood vessels which supply blood to the subject tissue and which result, inter alia, in lowered oxygen transport to such tissue, impaired tissue performance, tissue dysfunction and/or necrosis. Alternatively, where blood flow or organ perfusion may be quantitatively adequate, the oxygen carrying capacity of the blood or organ perfusion medium may be reduced, e.g., in hypoxic environment, such that oxygen supply to the tissue is lowered, and impaired tissue performance, tissue dysfunction, and/or tissue necrosis ensues.

The term "treating", "treat" or "treatment" as used herein includes preventative (e.g., prophylactic) and palliative treatment.

By "pharmaceutically acceptable" it is meant the carrier, diluent, excipients, and/or salt must be compatible with the other ingredients of the formulation, and not deleterious to the recipient thereof.

As used herein, the expressions "reaction-inert solvent" and "inert solvent" refers to a solvent or mixture of solvents which does not interact with starting materials, reagents, intermediates or products in a manner which adversely affects the yield of the desired product.

It will be recognized that the compound of this invention can exist in radiolabelled form, i.e., the compound may contain one or more atoms containing an atomic mass or mass number different from the atomic mass or mass number ordinary found in nature. Radioisotopes of hydrogen, carbon, phosphorous, fluorine and chlorine include ³H, ¹⁴C, ³²P, ³⁵S, ¹⁸F and ³⁶Cl, respectively. A compound of this invention, which contains those radioisotopes and/or other radioisotopes of other atoms are within the scope of this invention. Tritiated, i.e., ³H, and carbon-14, i.e., ¹⁴C, radioisotopes are particularly preferred for their ease of preparation and detectability. A radiolabelled Formula I compound can generally be prepared by methods well known to those skilled in the art. Conveniently, such radiolabelled compounds can be prepared by carrying out the procedures disclosed in the Schemes and/or in the Examples below by substituting a readily available radiolabelled reagent for a non-radiolabelled reagent.

Other features and advantages will be apparent from the specification and claims which describe the invention.

### Brief Description of the Drawings

FIG. 1 is a characteristic x-ray powder diffraction pattern showing that Form A anhydrous N-(5-cyclopropyl-1-quinolin-5-yl-1H-pyrazole-4-carbonyl)-guanidine, monomesylate salt is crystalline. (Vertical Axis: Intensity (CPS); Horizontal Axis: Two theta (degrees))
FIG. 2 is a characteristic x-ray powder diffraction pattern showing that Form D anhydrous N-(5-cyclopropyl-1-quinolin-5-yl-1H-pyrazole-4-carbonyl)-guanidine, monomesylate salt is crystalline. (Vertical Axis: Intensity (CPS); Horizontal Axis: Two theta (degrees))
FIG. 3 is the characteristic x-ray powder diffraction pattern of the Form C, hemihydrate N-(5-cyclopropyl-1-quinolin-5-yl-1H-pyrazole-4-carbonyl)-guanidine, monomesylate salt. (Vertical Axis: Intensity (CPS); Horizontal Axis: Two theta (degrees))

### DETAILED DESCRIPTION OF THE INVENTION

In general the crystals of this invention can be made by processes which include processes analogous to those known in the chemical arts, particularly in light of the description contained herein. Certain processes for the manufacture of the crystals of this invention are provided as further features of the invention and are illustrated by the following reaction scheme. Other processes are described in the experimental section.

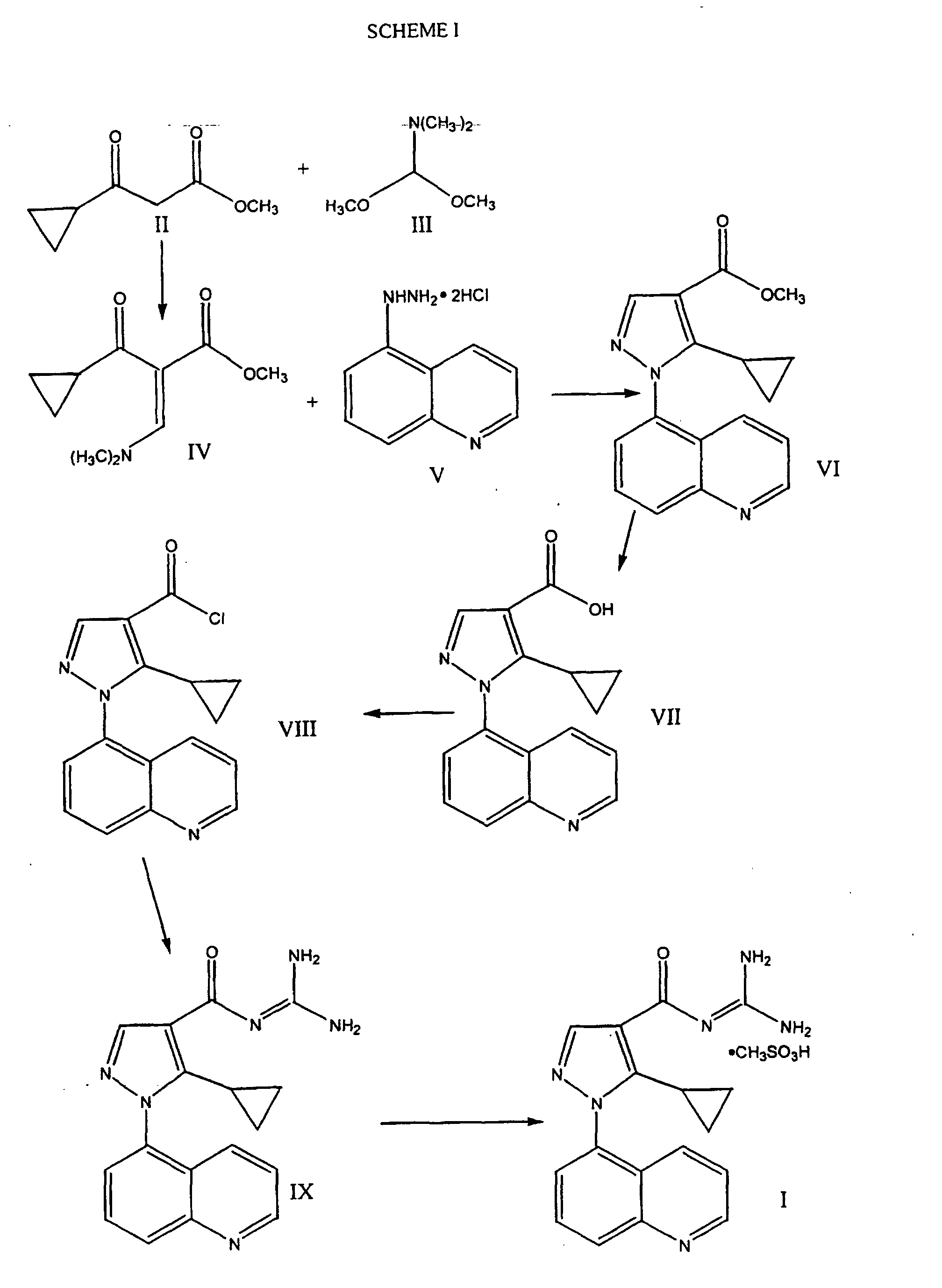

According to Scheme I the Formula II compound is combined with excess Formula III compound, (N,N-dimethyl amide dimethyl acetal), optionally, in the presence of an acid catalyst such as *p*-toluenesulfonic acid, under neat conditions at a temperature of about 50°C to about 110°C for about one to about five hours, preferably at a temperature of about 70°C to about 80°C for about one to about two hours to prepare the Formula IV compound. This reaction can be performed in ethyl acetate as well.

The Formula IV compound is cyclized with a Formula V compound in an inert solvent such as ethanol, preferably in the presence of an amine base such as triethylamine at a temperature of about 50°C to about reflux (78°C) for about 1 hour to about four hours to form the Formula VI pyrazole compound. This reaction may also be performed in ethyl acetate and methanol.

The Formula VI pyrazole is hydrolyzed with a base such as sodium hydroxide in a solvent such as methanol conveniently at ambient temperature or preferably at elevated temperature (e.g., reflux) for about one hour to about five hours to prepare the Formula VII acid.

Generally, the Formula VII acid is coupled with guanidine in the presence of a suitable coupling agent. A suitable coupling agent is one which transforms a carboxylic acid into a reactive species which forms an amide linkage on reaction with an amine.

The coupling agent may be a reagent which effects this condensation in a one pot process when mixed together with the carboxylic acid and guanidine. Exemplary coupling reagents are 1-(3-dimethylaminopropyl)-3-ethylcarbodiimide hydrochloride-hydroxybenzotriazole (EDC/HOBT), dicyclohexylcarbodiimide/hydroxybenzotriazole(HOBT), 2-ethoxy-1-ethoxycarbonyl-1,2-dihydroquinoline (EEDQ), and diethylphosphorylcyanide. The coupling is performed in an inert solvent, preferably an aprotic solvent at a temperature of about -20°C to about 50°C for about 1 to about 48 hours, in the presence of excess guanidine as base. Exemplary solvents include acetonitrile, dichloromethane, dimethylformamide and chloroform or mixtures thereof.

The coupling agent can be an agent which converts the carboxylic acid to an activated intermediate which is isolated and/or formed in a first step and allowed to react with guanidine in a second step. Examples of such coupling agents and activated intermediates are thionyl chloride or oxalyl chloride to form the acid chloride, cyanuric fluoride to form an acid fluoride or an alkyl chloroformate such as isobutyl or isopropenyl chloroformate or propanephosphonic anhydride (propanephosphonic acid anhydride, PPA) (with a tertiary amine base) to form a mixed anhydride of the carboxylic acid, or carbonyldiimidazole to form an acylimidazole. If the coupling agent is oxalyl chloride, it is advantageous to employ a small amount of dimethylformamide as cosolvent with another solvent (such as dichloromethane) to catalyze the formation of the acid chloride. This activated acid derivative may be coupled by mixing with the intermediate in an appropriate solvent together with an appropriate base. Appropriate solvent/base combinations are, for example, dichloromethane, dimethylformamide or acetonitrile or mixtures thereof in the presence of excess guanidine as base. Other appropriate solvent/base combinations include water or a ((C₁-C₅)alcohol) or a mixture thereof together with a cosolvent such as dichloromethane, tetrahydrofuran or dioxane and a base such as sodium potassium or lithium hydroxide in sufficient quantity to consume the acid liberated in the reaction. Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Theime Verlag, 1974, Stuttgart; M. Bodansky, Principles of Peptide Synthesis, Springer-Verlag, Berlin 1984; and The Peptides, Analysis, Synthesis and Biology (ed. E. Gross and J. Meienhofer), vols 1-5 (Academic Press, NY 1979-1983).

In a preferred embodiment, the Formula VII acid is activated with an excess of thionyl chloride (e.g., 3 to 6 equivalents) in an aprotic solvent such as toluene at a temperature of about 60°C to about 90°C for about fifteen minutes to about two hours, preferably at a temperature of about 75°C for about one to two hours.

The resulting Formula VIII activated acid chloride in anhydrous tetrahydrofuran is combined with excess guanidine hydrochloride and an aqueous solution of an inorganic base (e.g., sodium hydroxide) in anhydrous tetrahydrofuran at a temperature of about -20°C to about 10°C for about one hour to about three hours with warming to ambient temperature over the last hour to prepare the Formula IX compound.

The Formula IX compound is combined with methanesulfonic acid in an aprotic solvent, preferably a mixture of acetone and 1-methyl-2-pyrrolidinone, preferably about 90% to about 60% acetone, the remainder 1-methyl-2-pyrrolidinone, at a temperature of about 40°C to about 80°C for about 10 minutes to about one hour followed by stirring at a temperature of about 20°C to about 30°C for about 3 hours to about 6 hours, preferably at a temperature of about ambient for about 5 hours in the absence of light. Preferably the solids are reslurried in acetone for about 6 to about 18 hours. The salt formation can also be performed in tetrahydrofuran. With this choice of solvent, a 95% ethanol reslurry is preferred.

The starting materials and reagents for the above described compounds, are also readily available or can be easily synthesized by those skilled in the art using conventional methods of organic synthesis. For example, many of the compounds used herein are related to, or are derived from compounds found in nature, in which there is a large scientific interest and commercial need, and accordingly many such compounds are commercially available or are reported in the literature or are easily prepared from other commonly available substances by methods which are reported in the literature.

An anhydrous crystalline form (D) of the compound of this invention may be prepared by combining an acetone solution of N-(5-cyclopropyl-1-quinolin-5-yl-1H-pyrazole-4-carbonyl)-guanidine and N-methyl pyrrolidinone with methanesulfonic acid at a temperature of about 30°C to about 60°C, preferably about 50°, for about 1 to about 10 hours, typically followed by agitation with cooling to a temperature of about 5°C, preferably less than 10°C. Alternative solvents include organic solvents such as methanol, ethanol and isopropanol. As appropriate, the product is filtered and dried under vacuum at 55°C-60°C for 24 to 72 hours until the solvent content is less than or equal to 0.5% by gas chromatograph.

An anhydrous crystalline form (A) of the compound of this invention may be prepared from the above Form D crystalline form by repulping (i.e., mixing a suspension of partially dissolved material) from acetone at a temperature of about 20°C to about 25°C, preferably ambient temperature, with agitation, for about 2 hours to about 24 hours, typically followed by drying at a temperature of about 30°C to a temperature of about 60°C. Alternative solvents include inert solvents such as acetonitrile, ethylacetate and tetrahydrofuran.

A hemihydrate crystalline form (C) of the compound of this invention may be prepared from a 90% pure Form A (likely containing N-(5-cyclopropyl-1-quinolin-5-yl-1H-pyrazole-4-carbonyl-guaridine, dimesylate salt as an impurity) by repulping in ethanol or isopropanol/water at a temperature of about 20°C to about 25°C, preferably ambient temperature for about 2 hours to about 24 hours, preferably with agitation. Typically the range is about 85% to about 95% ethanol and about 5% to about 15% water. Preferably the ratio is 91% to 9% ethanol/water. Alternative solvents include organic/water solvents such as acetonitrile, acetone, tetrahydrodrofuran, ethylacetate, with 5% to 10% water.

The following Table 1 details important properties for four forms of: N-(5-cyclopropyl-1-quinolin-5-yl-1H-pyrazole-4-carbonyl)-guanidine, monomesylate salt: amorphous; the two anhydrous crystalline forms (A and D); and the hemihydrate crystalline form.

**TABLE 1**

| Form | Melting Point (onset) | Habit | Comment |
|---|---|---|---|
| Amorphous | | Amorphous | |
| A; Ex. 6 | 228°C | Equant | Most Stable Anhydrous Form |
| D; Ex. 5 | 215°C | Equant | Stable Anhydrous Form |
| C; Ex. 7 | 140-170°C (desolvation) 209°C | Equant | Hemihydrate, Convert to Form D upon dehydration |

Those skilled in the art will recognize that the Formula I salt can exist in several tautomeric forms. All such tautomeric forms are considered as part of this invention. For example, all of the tautomeric forms of the carbonylguanidine moiety of the Formula I salt are included in this invention.

Any aldose reductase inhibitor may be used as the second compound (active agent) of this invention for combination therapies. The term aldose reductase inhibitor refers to compounds which inhibit the bioconversion of glucose to sorbitol catalyzed by the enzyme aldose reductase. Such inhibition is readily determined by those skilled in the art according to standard assays (J. Malone, Diabetes, 29:861-864, 1980. "Red Cell Sorbitol, an Indicator of Diabetic Control"). A variety of aldose reductase inhibitors are known to those skilled in the art.

An amount of the aldose reductase inhibitor that is effective for the activities of this invention may be used. Typically, an effective dosage for the aldose reductase inhibitors invention is in the range of about 0.1 mg/kg/day to 100 mg/kg/day in single or divided doses, preferably 0.1 mg/kg/day to 20 mg/kg/day in single or divided doses.

Any glycogen phosphorylase inhibitor may be used as the second compound of this invention. The term glycogen phosphorylase inhibitor refers to any substance or agent or any combination of substances and/or agents which reduces, retards, or eliminates the enzymatic action of glycogen phosphorylase. The currently known enzymatic action of glycogen phosphorylase is the degradation of glycogen by catalysis of the reversible reaction of a glycogen macromolecule and inorganic phosphate to glucose-1-phosphate and a glycogen macromolecule which is one glucosyl residue shorter than the original glycogen macromolecule (forward direction of glycogenolysis). Such actions are readily determined by those skilled in the art according to standard assays. A variety of these compounds are included in the following published international patent applications: PCT application publication WO 96/39384 and WO96/39385. However, other glycogen phosphorylase inhibitors will be known to those skilled in the art.

In general an effective dosage of the glycogen phosphorylase inhibitor for the pharmacological combination compositions of this invention, for example the ischemic damage reducing activities of combinations containing the glycogen phosphorylase inhibitor compounds of this invention, is in the range of 0.005 to 50 mg/kg/day, preferably 0.01 to 25 mg/kg/day and most preferably 0.1 to 15 mg/kg/day.

Those skilled in the art will recognize that other cardiovascular agents, for example, β-blockers (e.g., acebutolol, atenolol, bopindolol, labetolol, mepindolol, nadolol, oxprenol, pindolol, propranolol, sotalol), calcium channel blockers (e.g., amlodipine, nifedipine, nisoldipine, nitrendipine, verapamil), ACE inhibitors (e.g., captopril, enalapril), nitrates (e.g., isosorbide dinitrate, isosorbide 5-mononitrate, glyceryl trinitrate), diuretics (e.g., hydrochlorothiazide, indapamide, piretanide, xipamide), glycosides (e.g., digoxin, metildigoxin), thrombolytics (e.g. tPA), platelet inhibitors (e.g., reopro), aspirin, dipyridamol, potassium chloride, clonidine, prazosin, aldose reductase inhibitors (e.g., zopolrestat) and adenosine A₃ receptor agonists may be used in conjunction with the crystals of this invention.

The crystals of the present invention inhibit the sodium/proton (Na+/H+) exchange transport system and hence are useful as a therapeutic or prophylactic agent for diseases caused by the acceleration of the sodium/proton (Na+/H+) exchange transport system, for example, cardiovascular diseases [e.g., arteriosclerosis, hypertension, arrhythmia (e.g., ischemic arrhythmia, arrhythmia due to myocardial infarction, arrhythmia after PTCA or after thrombolysis, etc.), angina pectoris, cardiac hypertrophy, myocardial infarction, heart failure (e.g., congestive heart failure, acute heart failure, cardiac hypertrophy, etc.), restenosis after PTCA, shock (e.g. hemorrhagic shock, endotoxin shock, etc.)], renal diseases (e.g., diabetes mellitus, diabetic nephropathy, ischemic acute renal failure, etc.) organ disorders associated with ischemia or ischemic reperfusion [(e.g., heart muscle ischemic reperfusion associated disorders, acute renal failure, or disorders induced by surgical treatment such as coronary artery bypass grafting (CABG) surgeries, vascular surgeries, organ transplantation, non-cardiac surgeries or percutaneous transluminal coronary angioplasty (PTCA)], cerebrovascular diseases (e.g., ischemic stroke, hemorrhagic stroke, etc.), cerebro ischemic disorders (e.g., disorders associated with cerebral infarction, disorders caused after cerebral apoplexy as sequelae, or cerebral edema). The crystals of this invention can also be used as an agent for myocardial protection during coronary artery bypass grafting (CABG) surgeries, vascular surgeries, percutaneous transluminal coronary angioplasty (PTCA), organ transplantation, or non-cardiac surgeries.

Preferably, the crystals of this invention can be used as agents for myocardial protection before, during, or after coronary artery bypass grafting (CABG) surgeries, vascular surgeries, percutaneous transluminal coronary angioplasty (PTCA), organ transplantation, or non-cardiac surgeries.

Preferably, the crystals of this invention can be used as agents for myocardial protection in patients presenting with ongoing cardiac (acute coronary syndromes, e.g., myocardial infarction or unstable angina) or cerebral ischemic events (e.g., stroke).

Preferably, the crystals of this invention can be used as agents for chronic myocardial protection in patients with diagnosed coronary heart disease (e.g., previous myocardial infarction or unstable angina) or patients who are at high risk for myocardial infarction (e.g., age greater than 65 and two or more risk factors for coronary heart disease).

In addition to this, the. crystals of this invention are notable for their strong inhibitory effect on the proliferation of cells, for example the proliferation of fibroblast cells and the proliferation of the smooth muscle cells of the blood vessels. For this reason, the crystals of this invention are valuable therapeutic agents for use in diseases in which cell proliferation represents a primary or secondary cause and may, therefore, be used as antiatherosclerotic agents, and as agents against diabetic late complications, cancerous diseases, fibrotic diseases such as pulmonary fibrosis, hepatic fibrosis or renal fibrosis, glomerular nephrosclerosis, organ hypertrophies or hyperplasias, in particular hyperplasia or hypertrophy of the prostate, pulmonary fibrosis, diabetic complications or recurrent stricture after PTCA, or diseases caused by endothelial cell injury.

The utility of the crystals of the present invention as medical agents in the treatment of diseases, such as are detailed herein in mammals (e.g., humans) for example, myocardial protection during surgery or mycardial protection in patients presenting with ongoing cardiac or cerebral ischemic events or chronic cardioprotection in patients with diagnosed coronary heart disease, is demonstrated by the activity of the crystals of this invention in conventional preclinical cardioprotection assays [see the in vivo assay in Klein, H. et al., Circulation 92:912-917 (1995); the isolated heart assay in Scholz, W. et al., Cardiovascular Research 29:260-268 (1995); the antiarrhythmic assay in Yasutake M. et al., Am. J. Physiol., 36:H2430-H2440 (1994); the NMR assay in Kolke et al., J. Thorac. Cardiovasc. Surg. 112: 765-775 (1996)]. Such assays also provide a means whereby the activities of the crystals of this invention can be compared with the activities of other known crystals. The results of these comparisons are useful for determining dosage levels in mammals, including humans, for the treatment of such diseases.

Administration of the crystals of this invention can be via any method which delivers a crystal of this invention preferentially to the desired tissue (e.g., liver and/or cardiac tissues). These methods include oral routes, parenteral, intraduodenal routes, etc. Generally, the crystals of the present invention are administered in single (e.g., once daily) or multiple doses or via constant infusion in, for example, an isotonic saline solution.

The crystals of this invention are useful, for example, in reducing or minimizing damage effected directly to any tissue that may be susceptible to ischemia/reperfusion injury (e.g., heart, brain, lung, kidney, liver, gut, skeletal muscle, retina) as the result of an ischemic event (e.g., myocardial infarction). The active compound is therefore usefully employed prophylactically to prevent, i.e. (prospectively or prophylactically) to blunt or stem, tissue damage (e.g., myocardial tissue) in patients who are at risk for ischemia (e.g., myocardial ischemia).

Generally, the crystals of this invention are administered orally, or parenterally (e.g., intravenous, intramuscular, subcutaneous or intramedullary). Topical administration may also be indicated, for example, where the patient is suffering from gastrointestinal disorders or whenever the medication is best applied to the surface of a tissue or organ as determined by the attending physician.

The amount and timing of crystals administered will, of course, be dependent on the subject being treated, on the severity of the affliction, on the manner of administration and on the judgement of the prescribing physician. Thus, because of patient to patient variability, the dosages given below are a guideline and the physician may titrate doses of the drug to achieve the treatment that the physician considers appropriate for the patient. In considering the degree of treatment desired, the physician must balance a variety of factors such as age of the patient, presence of preexisting disease, as well as presence of other diseases (e.g., cardiovascular disease).

Thus, for example, in one mode of administration the crystals of this invention may be administered just prior to cardiac surgery (e.g., within twenty-four hours before surgery), during or subsequent to cardiac surgery (e.g., within twenty-four hours after surgery) where there is risk of myocardial ischemia. In an especially preferred mode an infusion is administered with a loading dose of about 1 mg to about 300 mg for about one minute to about one hour prior to surgery followed by a constant infusion of about 1 mg/kg/day to about 100 mg/kg/day for the remaining presurgery, surgery and post surgery periods, including for example about 2 to about 7 days post surgical treatment. The compounds of this invention may also be administered in a chronic daily mode.

An amount of the crystals of this invention is used that is effective for ischemic protection. A preferred dosage is about 0.001 to 100 mg/kg/day of the crystal of this invention. An especially preferred dosage is about 0.01 to 50 mg/kg/day of the crystal of this invention.

The crystals of the present invention are generally administered in the form of a pharmaceutical composition comprising at least one of the crystals of this invention together with a pharmaceutically acceptable vehicle or diluent. Thus, the crystals of this invention can be administered individually or together in any conventional oral, parenteral, rectal or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions, for example, in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

Pharmaceutical compositions according to the invention may contain for example 0.0001%-95% of the compound(s) of this invention. In any event, the composition or formulation to be administered will contain a quantity of a crystals(s) according to the invention in an amount effective to treat the disease/condition of the subject being treated.

The crystals of this invention generally will be administered in a convenient formulation. The following formulation examples are illustrative only and are not intended to limit the scope of the present invention.

In the formulations which follow, "active ingredient" means a compound(s) (crystals(s)) of this invention.

### Formulation 1: Gelatin Capsules

Hard gelatin capsules are prepared using the following:

| Ingredient | Quantity (mg/capsule) |
|---|---|
| Active ingredient | 0.25-100 |
| Starch, NF | 0-650 |
| Starch flowable powder | 0-50 |
| Silicone fluid 350 centistokes | 0-15 |

A tablet formulation is prepared using the ingredients below:

### Formulation 2: Tablets

| Ingredient | Quantity (mg/tablet) |
|---|---|
| Active ingredient | 0.25-100 |
| Cellulose, microcrystalline | 200-650 |
| Silicon dioxide, fumed | 10-650 |
| Stearate acid | 5-15 |

The components are blended and compressed to form tablets.

Alternatively, tablets each containing 0.25-100 mg of active ingredients are made up as follows:

### Formulation 3: Tablets

| Ingredient | Quantity (mg/tablet) |
|---|---|
| Active ingredient | 0.25-100 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50° - 60°C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.25-100 mg of active ingredient per 5 ml dose are made as follows:

### Formulation 4: Suspensions

| Ingredient | Quantity (mg/5 ml) |
|---|---|
| Active ingredient | 0.25-100 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified Water to | 5 mL |

The active ingredient is passed through a No. 45 mesh U.S. sieve and mixed with the sodium carboxymethyl cellulose and syrup to form smooth paste. The benzoic acid solution, flavor, and color are diluted with some of the water and added, with stirring. Sufficient water is then added to produce the required volume. An aerosol solution is prepared containing the following ingredients:

### Formulation 5: Aerosol

| Ingredient | Quantity (% by weight) |
|---|---|
| Active ingredient | 0.25 |
| Ethanol | 25.75 |
| Propellant 22 (Chlorodifluoromethane) | 74.00 |

The active ingredient is mixed with ethanol and the mixture added to a portion of the propellant 22, cooled to 30°C, and transferred to a filling device. The required amount is then fed to a stainless steel container and diluted with the remaining propellant. The valve units are then fitted to the container. Suppositories are prepared as follows:

### Formulation 6: Suppositories

| Ingredient | Quantity (mg/suppository) |
|---|---|
| Active ingredient | 250 |
| Saturated fatty acid glycerides | 2,000 |

The active ingredient is passed through a No. 60 mesh U.S. sieve and suspended in the saturated fatty acid glycerides previously melted using the minimal necessary heat. The mixture is then poured into a suppository mold of nominal 2 g capacity and allowed to cool.

An intravenous formulation is prepared as follows:

### Formulation 7: Intravenous Solution

| Ingredient | Quantity |
|---|---|
| Active ingredient | 25 mg |
| Isotonic saline | 1,000 mL |

The solution of the above ingredients is intravenously administered to a patient.

The active ingredient above may also be a combination of agents.

### EXAMPLE 1

Methyl-3-cyclopropyl-3-oxopropanoate (15 g, 106 mmol, 1 equiv) and *N*,*N*-dimethylformamide dimethylacetal (14.7 mL, 111 mmol, 1.05 equiv) were heated at 75 °C for 1.5 h under N₂. The orange oil was then cooled to room temperature. TLC analysis (1:1 EtOAc/hexanes) indicated disappearance of starting material and appearance of a minor less polar spot and a major more polar spot (methyl-3-cyclopropyl-2-dimethylenamino-3-oxopropanoate). The crude mixture was used as is in the next step.

### EXAMPLE 2

Crude methyl-3-cyclopropyl-2-dimethylenamino-3-oxopropanoate (20.9 g, 106 mmol, 1.07 equiv) was diluted with ethanol (250 mL). Triethylamine (34.4 mL, 247 mmol, 2.5 equiv) followed by quinolin-5-yl-hydrazine (22.9 g, 98.6 mmol, 1 equiv) was added sequentially. Slight gas evolution upon addition of quinolin-5-yl-hydrazine was observed. The resulting heterogeneous mixture was heated at reflux (78 °C) under N₂ for 2 h. The mixture became homogeneous and very dark after about 3 min of heating. The mixture was then cooled to room temperature. TLC analysis (1:1 EtOAc/hexanes) indicated a slightly less polar spot (5-cyclopropyl-1-quinolin-5-yl-1H-pyrazole-4- carboxylic acid methyl ester). APCI mass spec indicated desired product as well. The reaction mixture was then concentrated. To the residue was added EtOAc (300 mL) and 0.1 N HCl (400 mL). This emulsion was stirred for 10 min at room temperature and then filtered through a pad of Celite® to remove solids. The resulting biphasic mixture was separated. The aqueous layer was extracted with EtOAc (2 X 300 mL). The combined organic layers were washed with 0.1N HCl (2 X 300 mL), then dried over sodium sulfate, and concentrated. To the residue was added hot isopropyl ether (80 mL). The cloudy solution was stirred for 2 min. Then hexanes (125 mL) were added. The solids were allowed to granulate overnight. Solids were collected by filtration to provide the product, 5-cyclopropyl-1-quinolin-5-yl-1H-pyrazole-4-carboxylic acid methyl ester, as a yellow orange powder (20.8 g, 72% over 2 steps).

### EXAMPLE 3

To a solution of 5-cyclopropyl-1-quinolin-5-yl-1H-pyrazole-4-carboxylic acid methyl ester (20 g, 68.2 mmol, 1 equiv) in MeOH (120 mL) was added 2N NaOH (54.5 mL, 109 mmol, 1.6 equiv). The resulting solution was heated at reflux (65 °C) for 1.5 h under N₂, and then allowed to cool to room temperature. TLC analysis (1:1 EtOAc/hexanes) indicated disappearance of starting material. The methanol was removed under vacumn with gentle heating (35 °C) on a rotovap. The basic aqueous layer was then washed with EtOAc (2 X 100 mL). The resulting basic aqueous layer was acidified slowly to pH 1-2 with concentrated HCl. The product precipitated out during acidification. The slurry was stirred at room temperature for 0.5 h, then the solids were collected by filtration. The solids were washed with 1N HCl (2 X 25 mL) and dried to afford the acid as a pale brown solid (18.8 g, 99%).

### EXAMPLE 4

To a stirred suspension of 5-cyclopropyl-1-quinolin-5-yl-1H-pyrazole-4-carboxylic acid (25 g, 89.5 mmol, 1 equiv) in toluene (250 mL) was added thionyl chloride (32.6 mL, 448 mmol, 5 equiv). The resulting suspension was heated at 75 °C for 1.5 h under N₂. The reaction mixture stayed heterogeneous throughout. The solid acid chloride was collected by filtration. The tan solid was washed with toluene (3 X 50 mL) and dried under vacumn.

A suspension of the acid chloride in THF (250 mL) was cooled to 0 °C. A solution of guanidine hydrochloride (17.1g, 179 mmol, 2 equiv) and 2N NaOH (224 mL, 448 mmol, 5 equiv) was added via a dropping addition funnel over 5-10 min under N₂. The reaction became homogenous and biphasic upon addition of the basic aqueous solution of guanidine. The mixture was stirred at 0 °C with slow warming over 1 h to room temperature and then for an additional 1 h at room temperature. TLC analysis (4:1 dichloromethane/methanol) indicated appearance of a more polar spot (N-(5-cyclopropyl-1-quinolin-5-yl-1H-pyrazole-4-carbonyl)-guanidine) and trace starting material acid. THF was removed under vacumn with gentle heating (35 °C) which resulted in precipitation of the product. The aqueous layer was stirred at room temperature for 1 h to allow the product to granulate. The solid was collected by filtration, washed with water (2 X 50 mL), and dried. The color of the product has ranges from off-white to medium brown. This batch was medium brown. Reslurry in MeOH (125 mL) for 30 min provided the desired product N-(5-cyclopropyl-1-quinolin-5-yl-1H-pyrazole-4-carbonyl)-guanidine (22.6 g, 79% yield) as a pale tan solid.

### EXAMPLE 5

N-(5-Cyclopropyl-1-quinolin-5-yl-1H-pyrazole-4-carbonyl)-guanidine (3.08 kg, 9.61 mol, 1 equiv) was suspended in acetone (30.8 kg). 1-Methyl-2-pyrrolidinone (12.3 kg) was added to obtain a homogenenous solution. An additional 4.8 kg of acetone was used to rinse forward (spec. free filtration). The reaction solution was warmed to 50°C. A solution of methanesulfonic acid (0.83 kg, 8.65 mol, 0.9 equiv) in acetone (8.3 kg) was added while keeping the temperature below 55°C. The slurry that was obtained was agitated at 50°C for 1-2 hours, then cooled, and filtered. The filter cake was rinsed with acetone and then dried to afford N-(5-cyclopropyl-1-quinolin-5-yl-1H-pyrazole-4-carbonyl)-guanidine, monomesylate salt (3.24 kg, 81 %) as an off-white solid. The product was then dried under vacuum affording an anhydrous crystal having the following properties (Form D).
Microscopy: birefringent plate/equant
Degree of Crystallinity: fully crystalline
Hygroscopicity: non-hygroscopic
Appearance: off white crystalline solid
Melting point: 215°C (onset temperature at 5°C/min)
The X-Ray diffraction d-spacing is provided in the following Table II

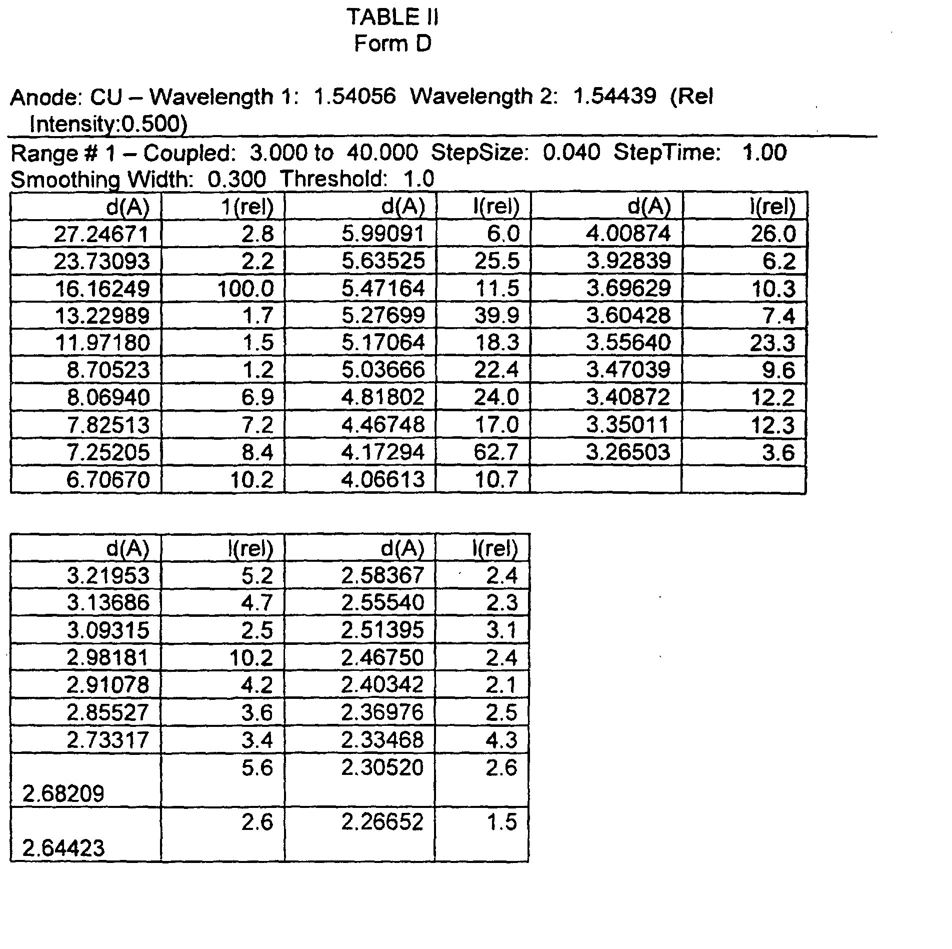

### EXAMPLE 6

To 3.165 kgs of the product of Example 5 was added 123 Liters (3.8 volumes) of acetone. The slurry was agitated for 20 hours at room temperature. The slurry was filtered, and solids were dried at 50°C. The product was an anhydrous crystal (3.145 kg, 99%) (Form A) having the following properties.
Microscopy: birefringent equant
Degree of Crystallinity: fully crystalline
Hygroscopicity: non-hygroscopic
Appearance: white crystalline solid
Melting point: 228°C (onset temperature at 5°C/min)
The X-Ray diffraction d-spacing is provided in the following Table III

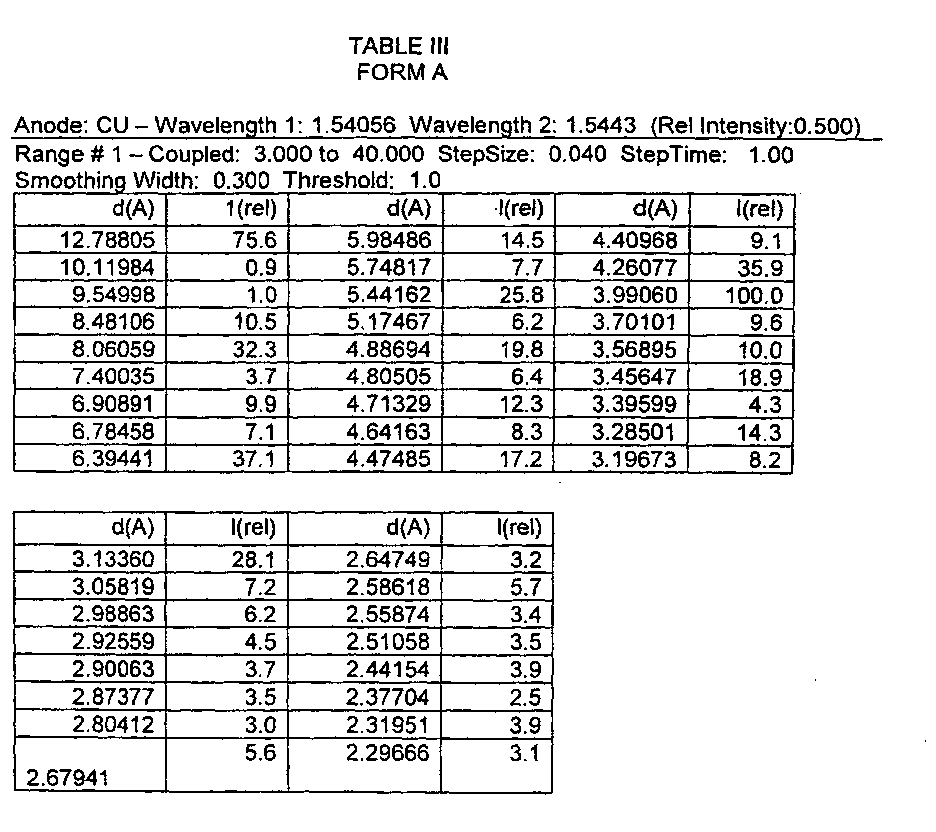

### EXAMPLE 7

To 0.25 gram of 1 ml of an approx. 90% pure sample of Form A (likely containing N-(5-cyclopropyl-1-quinolin-5-yl-1H-pyrazole-4-carbonyl)-guanidine, dimesylate as an impurity) ethanol was added (EtOH contained ∼9% water). The slurry was agitated for about 21 hours at room temperature. The slurry was then filtered, and the solid was dried at 50°C under vacuum for 90 minutes affording a hemihydrate crystal (Form C) having the following properties.
Microscopy: birefringent equant
Degree of Crystallinity: fully crystalline
Appearance: cream-colored crystalline solid
Melting point: 140°C - 170°C desolvation, 209°C (onset temperature at 5°C/min)
The X-Ray diffraction d-spacing is provided in the following Table IV

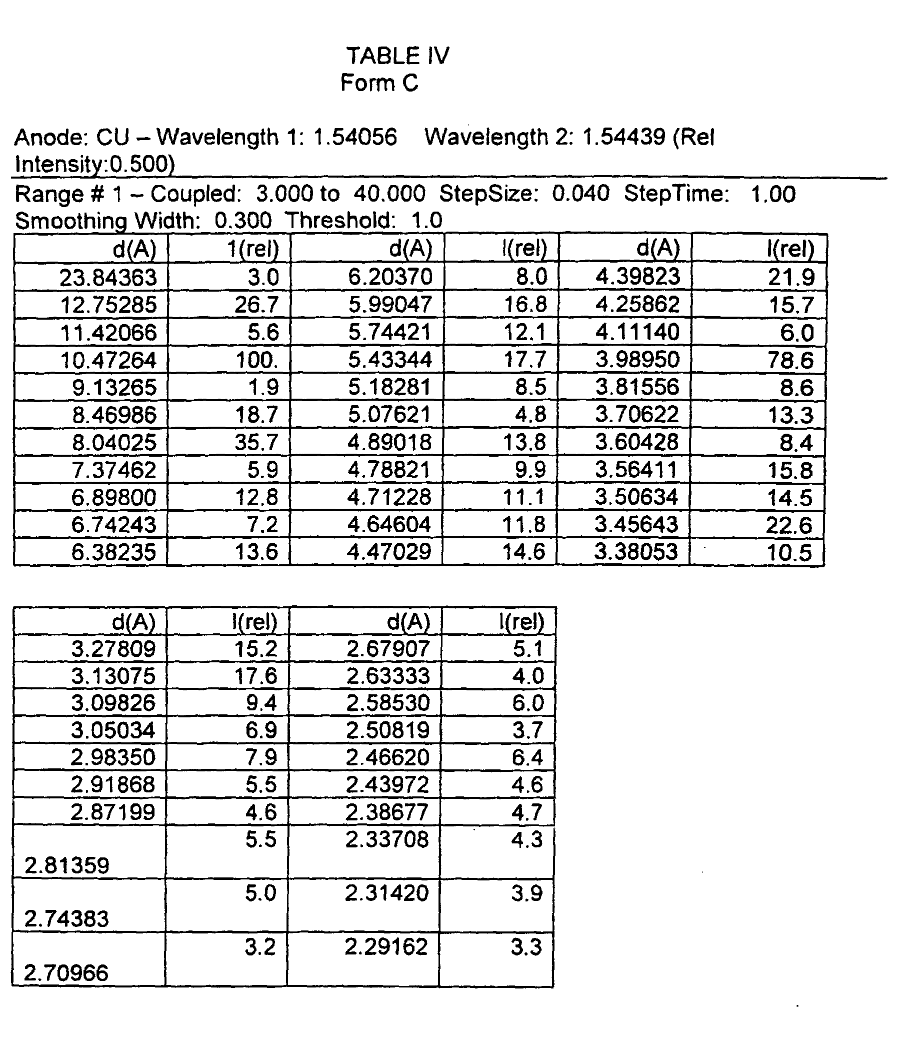

## Claims

1. A crystalline form of a salt having the Formula I

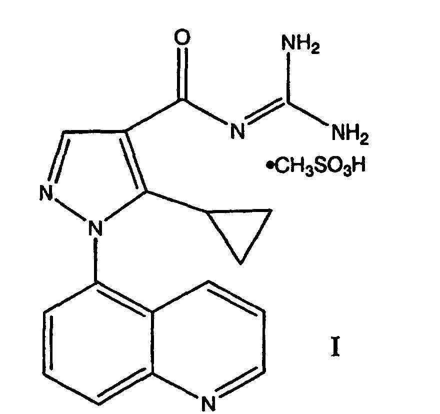

2. A crystal of claim 1 which is anhydrous N-(5-cyclopropyl-1-quinolin-5-yl-1H-pyrazole-4-carbonyl)-guanidine, monomesylate salt.

3. A crystal of claim 1 which is anhydrous N-(5-cyclopropyl-1-quinolin-5-yl-1H-pyrazole-4-carbonyl)-guanidine, monomesylate salt Form D having the following X-ray diffraction pattern

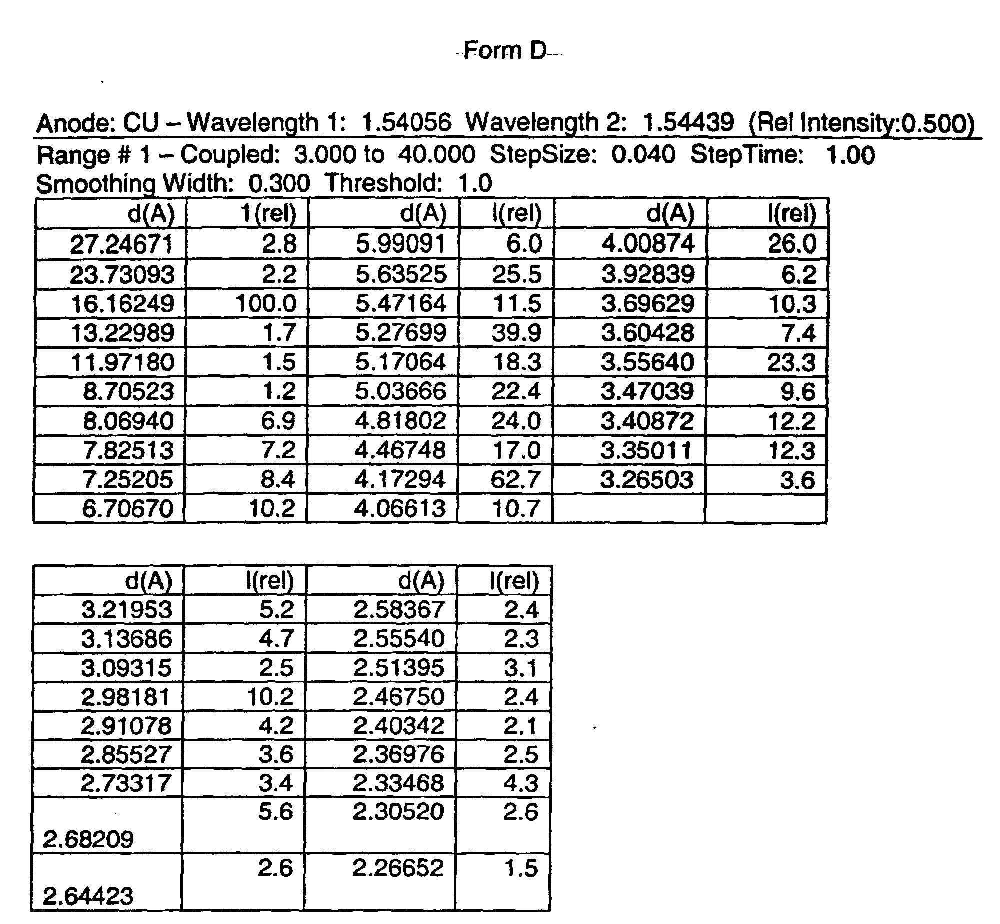

4. A crystal of claim 1 which is the anhydrous N-(5-cyclopropyl-1-quinolin-5-yl-1H-pyrazole-4-carbonyl)-guanidine, monomesylate salt Form A having the following X-ray diffraction pattern

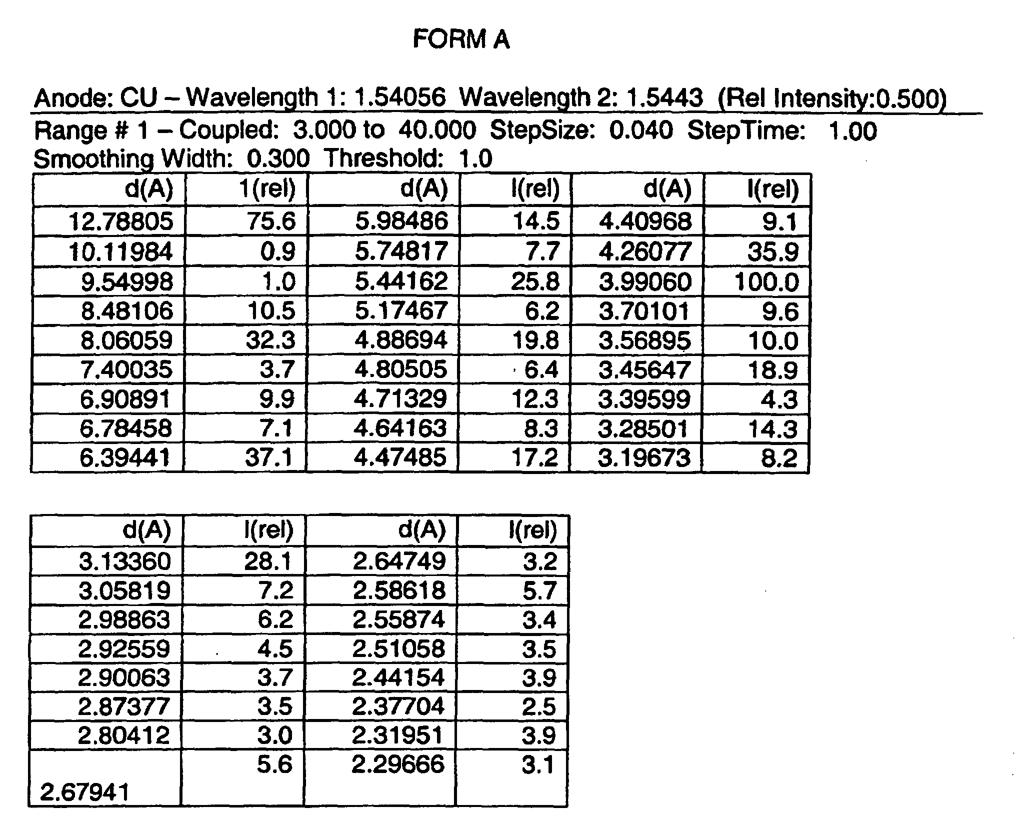

5. A crystal of claim 1 which is N-(5-cyclopropyl-1-quinolin-5-yl-1H-pyrazole-4-carbonyl)-guanidine, monomesylate hemihydrate.

6. A method for making the crystal of claim 3 comprising combining an acetone solution of N-(5-cyclopropyl-1-quinolin-5-yl-1H-pyrazole-4-carbonyl)-guanidine and N-methyl pyrrolidinone with methanesulfonic acid at a temperature of about 30°C to about 60°C, followed by cooling to about ambient temperature.

7. A method for making the crystal of claim 3 comprising combining an acetone solution of N-(5-cyclopropyl-1-quinolin-5-yl-1H-pyrazole-4-carbonyl)-guanidine and N-methyl pyrrolidinone with methanesulfonic acid at a temperature of about 30°C to about 60°C, followed by cooling to a temperature of less than about 10°C.

8. A method for making the crystal of claim 3 comprising combining an acetone solution of N-(5-cyclopropyl-1-quinolin-5-yl-1H-pyrazole-4-carbonyl)-guanidine and N-methyl pyrrolidinone with methanesulfonic acid at a temperature of about 30°C to about 60°C, followed by cooling to a temperature of about 5°C.

9. A method for making the crystal of claim 4 comprising repulping the Form D anhydrous crystal of claim 3 in acetone at a temperature of about 20°C to about 25°C.

10. A method for making the crystal of claim 5 comprising repulping the anhydrous Form A crystal of claim 4 in ethanol/water or iso-propanol/water at a temperature of about 20°C to about 25°C.

11. The method according to claim 8 wherein ethanol/water is used and the ratio is about 85% to about 95% ethanol and about 5% to about 15% water.

12. [5-Cyclopropyl-1-(quinolin-5-yl)-1H-pyrazole-4-carbonyl]guanidine, monomesylate hemihydrate.

13. The use of a crystal according to claim 1 for the preparation of a medicament for reducing tissue damage in a mammal resulting from ischemia or hypoxia

14. The use according to claim 13 wherein the tissue is cardiac, brain, liver, kidney, lung, gut, skeletal muscle, spleen, pancreas, nerve, spinal cord, retina tissue, the vasculature, or intestinal tissue.

15. The use according to claim 14 wherein the amount of the Formula I crystal is sufficient to provide a dose of about 0.01 mg/kg/day to about 50 mg/kg/day.

16. The use according to claim 15 wherein the mammal is a female or male human.

17. The use according to claim 16 wherein the medicament is to be administered prophylactically.

18. The use according to claim 16 wherein the medicament is to be administered prior to surgery.

19. The use according to claim 16 wherein the medicament is to be administered prior to cardiac surgery.

20. The use according to claim 16 wherein the medicament is to be administered during surgery.

21. The use according to claim 16 wherein the medicament is to be administered during cardiac surgery.

22. The use according to claim 16 wherein the medicament is to be administered within twenty-four hours after surgery.

23. The use according to claim 16 wherein the tissue damage resulting from ischemia is ischemic damage and is incurred during organ transplantation.

24. The use according to claim 16 wherein the medicament is to be administered to prevent perioperative myocardial ischemic injury.

25. A pharmaceutical composition which comprises a therapeutically effective amount of a crystal of claim 1 and a pharmaceutically acceptable carrier, vehicle or diluent.

26. A pharmaceutical composition for the reduction of tissue damage resulting from ischemia or hypoxia which comprises a therapeutically effective amount of a crystal of claim 1 and a pharmaceutically acceptable carrier, vehicle or diluent.

27. The use according to claim 16 wherein the medicament is to be administered prior to, during and after surgery.

28. The use according to claim 16 wherein the medicament is to be administered prior to, during and after cardiac surgery.

29. A crystal according to any of claims 1 to 5 for use as a pharmaceutical agent.

## Patentansprüche

1. Kristalline Form eines Salzes der Formel I

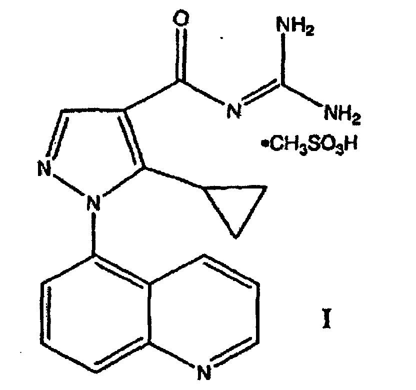

2. Kristall nach Anspruch 1, nämlich das wasserfreie Monomesylatsalz von N-(5-Cyclopropyl-1-chinolin-5-yl-1H-pyrazol-4-carbonyl)guanidin.

3. Kristall nach Anspruch 1, nämlich die Form D des wasserfreien Monomesylatsalzes von N-(5-Cyclopropyl-1-chinolin-5-yl-1H-pyrazol-4-carbonyl)guanidin mit dem folgenden Röntgenbeugungsspektrum.

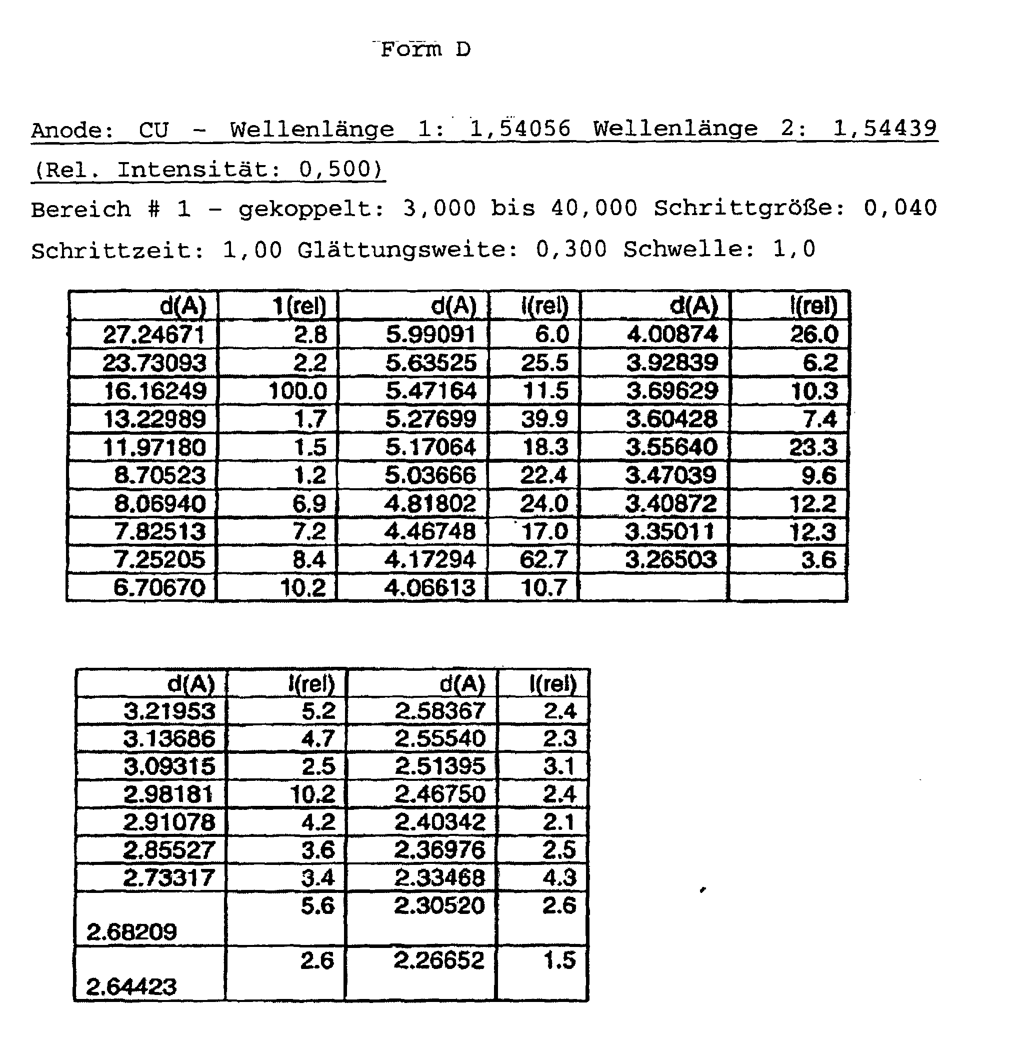

4. Kristall nach Anspruch 1, nämlich die Form A des wasserfreien Monomesylatsalzes von N-(5-Cyclopropyl-1-chinolin-5-yl-1H-pyrazol-4-carbonyl)guanidin mit dem folgenden Röntgenbeugungsspektrum.

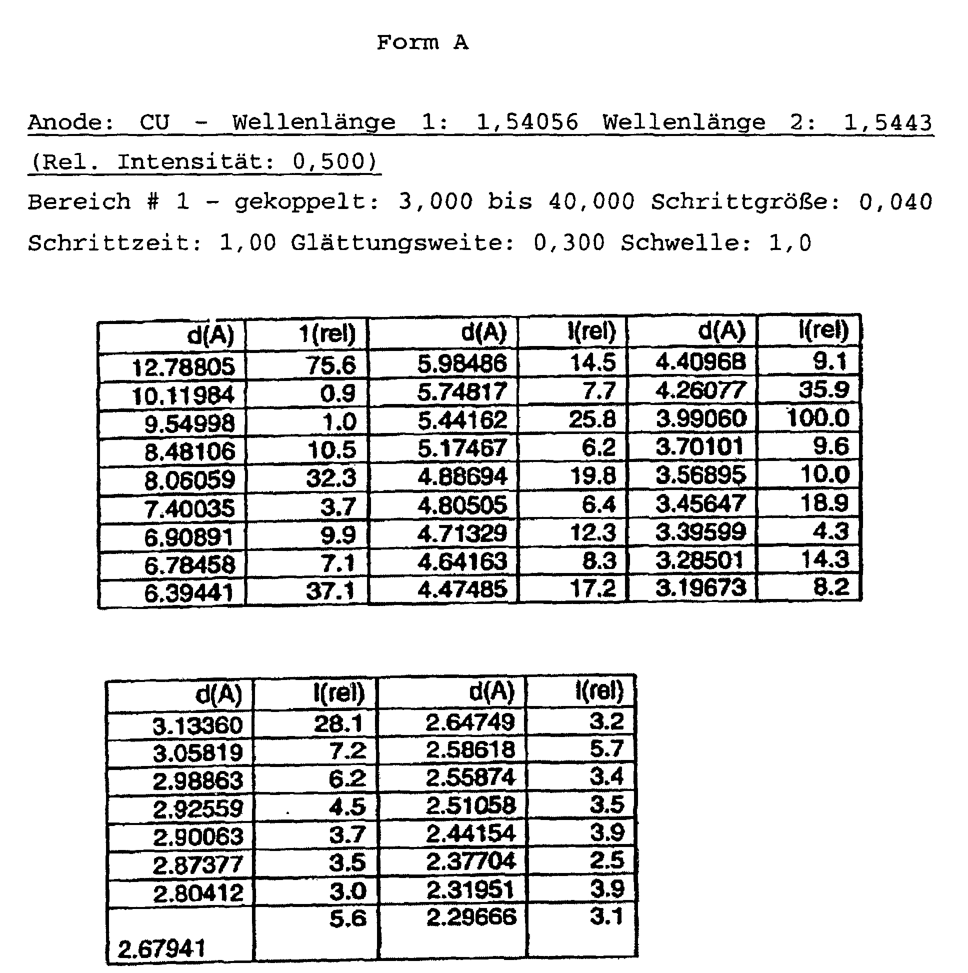

5. Kristall nach Anspruch 1, nämlich das Monomesylathemihydrat von N-(5-Cyclopropyl-1-chinolin-5-yl-1H-pyrazol-4-carbonyl) guanidin.

6. Verfahren zur Herstellung des Kristalls nach Anspruch 3, umfassend die Vereinigung einer Aceton-Lösung von N-(5-Cyclopropyl-1-chinolin-5-yl-2H-pyrazol-4-carbonyl)guanidin und N-Methylpyrrolidinon mit Methansulfonsäure bei einer Temperatur von etwa 30°C bis etwa 60°C und anschließendes Abkühlen auf etwa Umgebungstemperatur.

7. Verfahren zur Herstellung des Kristalls nach Anspruch 3, umfassend die Vereinigung einer Aceton-Lösung von N-(5-Cyclopropyl-1-chinolin-5-yl-1H-pyrazol-4-carbonyl)guanidin und N-Methylpyrrolidinon mit Methansulfonsäure bei einer Temperatur von etwa 30°C bis etwa 60°C und anschließendes Abkühlen auf eine Temperatur von weniger als etwa 10°C.

8. Verfahren zur Herstellung des Kristalls nach Anspruch 3, umfassend die Vereinigung einer Aceton-Lösung von N-(5-Cyclopropyl-1-chinolin-5-yl-1H-pyrazol-4-carbonyl)guanidin und N-Methylpyrrolidinon mit Methansulfonsäure bei einer Temperatur von etwa 30°C bis etwa 60°C und anschließendes Abkühlen auf eine Temperatur von etwa 5°C.

9. Verfahren zur Herstellung des Kristalls nach Anspruch 4, umfassend das Wiederaufschlämmen der wasserfreien Kristallform D nach Anspruch 3 in Aceton bei einer Temperatur von etwa 20°C bis etwa 25°C.

10. Verfahren zur Herstellung des Kristalls nach Anspruch 5, umfassend das Wiederaufschlämmen der wasserfreien Kristallform A nach Anspruch 4 in Ethanol/Wasser oder Isopropanol/Wasser bei einer Temperatur von etwa 20°C bis 25°C.

11. Verfahren nach Anspruch 8, worin Ethanol/Wasser verwendet wird und das Verhältnis etwa 85% bis etwa 95% Ethanol und etwa 5% bis etwa 15% Wasser beträgt.

12. Monomesylathemihydrat von [5-Cyclopropyl-1-(chinolin-5-yl)-1H-pyrazol-4-carbonyl]guanidin.

13. Verwendung eines Kristalls nach Anspruch 1 zur Herstellung eines Arzneimittels zur Verringerung von Gewebeschädigungen in einem Säugetier, die von Ischämie oder Hypoxie herrührt.

14. Verbindung nach Anspruch 13, worin das Gewebe Herz-, Hirn-, Leber-, Nieren-, Lungen-, Darm-, Skelettmuskel-, Milz-, Pankreas-, Nerven-, Rückenmark-, Retinagewebe, das Gefäßsystem oder Darmgewebe ist.

15. Verwendung nach Anspruch 14, worin die Menge des Kristalls der Formel I ausreichend ist für eine Dosis von etwa 0,01 mg/kg/Tag bis etwa 50 mg/kg/Tag.

16. Verwendung nach Anspruch 15, worin das Säugetier ein weiblicher oder männlicher Mensch ist.

17. Verwendung nach Anspruch 16, worin das Arzneimittel prophylaktisch verabreicht werden soll.

18. Verwendung nach Anspruch 16, worin das Arzneimittel vor einer Operation verabreicht werden soll.

19. Verwendung nach Anspruch 16, worin das Arzneimittel vor einer Herzoperation verabreicht werden soll.

20. Verwendung nach Anspruch 16, worin das Arzneimittel während der Operation verabreicht werden soll.

21. Verwendung nach Anspruch 16, worin das Arzneimittel während einer Herzoperation verabreicht werden soll.

22. Verwendung nach Anspruch 16, worin das Arzneimittel innerhalb von 24 Stunden nach der Operation verabreicht werden soll.

23. Verwendung nach Anspruch 16, worin der Gewebsschaden, der von Ischämie herrührt, ein ischämischer Schaden ist und während einer Organtransplantation auftritt.

24. Verwendung nach Anspruch 16, worin das Arzneimittel verabreicht werden soll, um eine perioperative myokardiale ischämische Verletzung zu verhindern.

25. Pharmazeutische Zusammensetzung, umfassend eine therapeutisch wirksame Menge eines Kristalls nach Anspruch 1 und einen pharmazeutisch annehmbaren Träger, Vehikel oder Verdünnungsmittel.

26. Pharmazeutische Zusammensetzung zur Verringerung von Gewebeschädigung, die von Ischämie oder Hypoxie herrührt, umfassend eine therapeutisch wirksame Menge eines Kristalls nach Anspruch 1 und einen pharmazeutisch annehmbaren Träger, Vehikel oder Verdünnungsmittel.

27. Verwendung nach Anspruch 16, worin das Arzneimittel vor, während und nach der Operation verabreicht werden soll.

28. Verwendung nach Anspruch 16, worin das Arzneimittel vor, während und nach einer Herzoperation verabreicht werden soll.

29. Kristall nach einem der Ansprüche 1 bis 5 zur Verwendung als pharmazeutisches Mittel.

## Revendications

1. Forme cristalline d'un sel répondant à la formule I

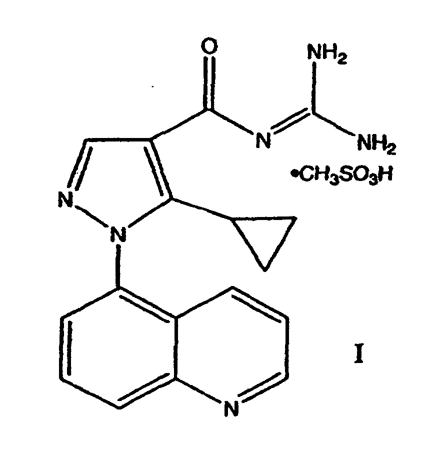

2. Forme cristalline suivant la revendication 1, qui est le sel monomésylate de N-(5-cyclopropyl-1-quinoléine-5-yl-1H-pyrazole-4-carbonyl)-guanidine anhydre.

3. Forme cristalline suivant la revendication 1, qui est la forme D de sel monomésylate de N-(5-cyclopropyl-1-quinoléine-5-yl-1H-pyrazole-4-carbonyl)-guanidine anhydre ayant le diagramme de diffraction des rayons X suivants

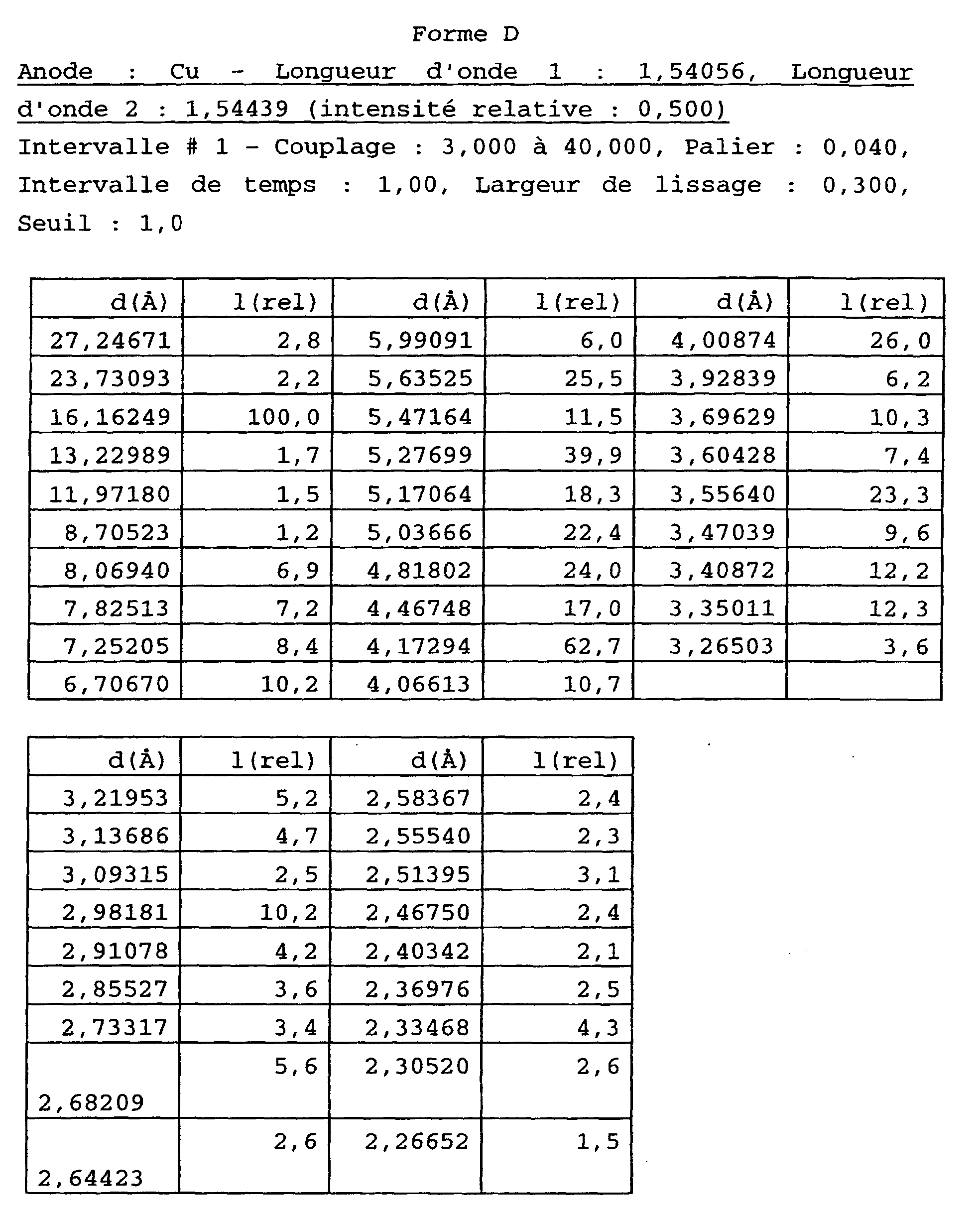

4. Forme cristalline suivant la revendication 1, qui est la forme A de sel monomésylate de N-(5-cyclopropyl-1-quinoléine-5-yl-1H-pyrazole-4-carbonyl)-guanidine anhydre ayant le diagramme de diffraction des rayons X suivants

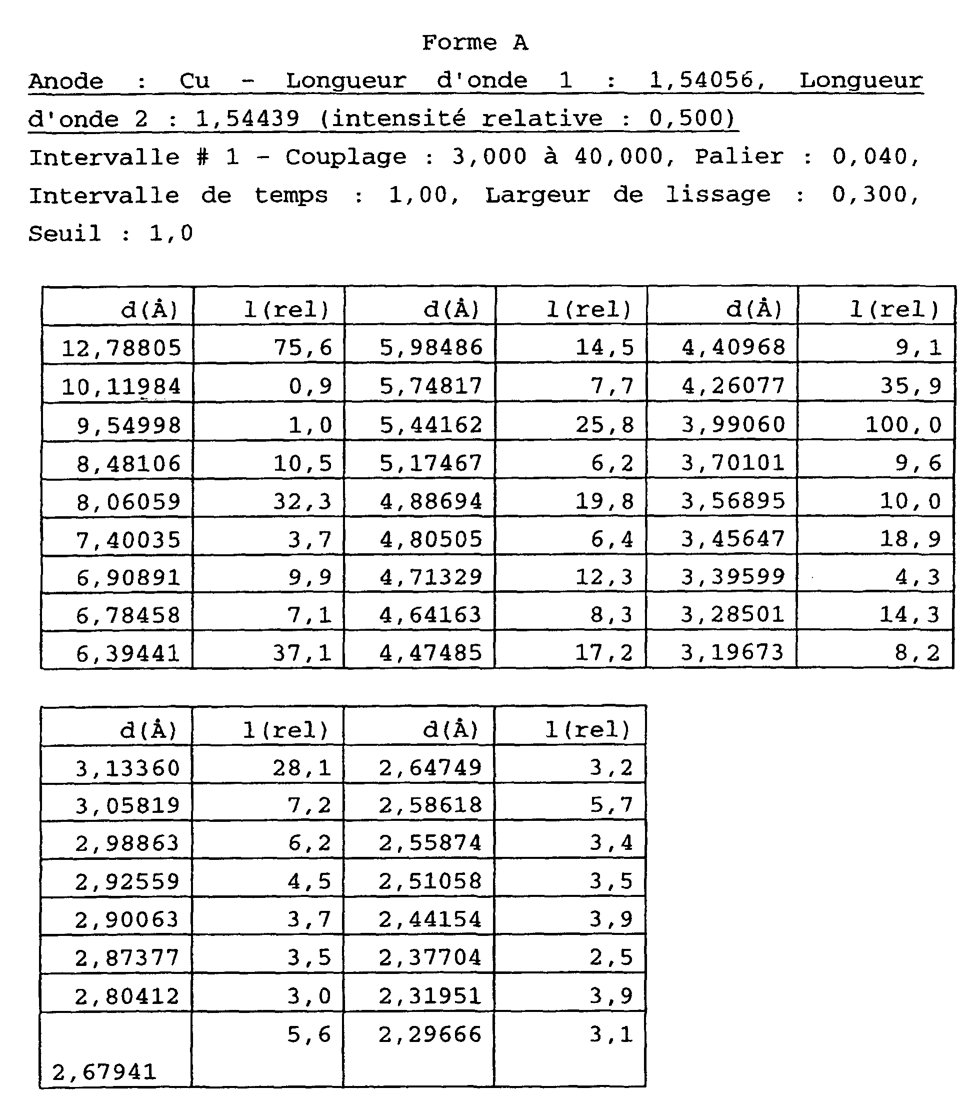

5. Forme cristalline suivant la revendication 1 qui est hémihydrate de monomésylate de N-(5-cyclopropyl-1-quinoléine-5-yl-1H-pyrazole-4-carbonyl)-guanidine.

6. Procédé pour la préparation de la forme cristalline de la revendication 3, comprenant l'association d'une solution acétonique de N-(5-cyclopropyl-1-quinoléine-5-yl-1H-pyrazole-4-carbonyl)-guanidine et de N-méthylpyrrolidinone avec de l'acide méthanesulfonique à une température comprise dans l'intervalle d'environ 30°C à environ 60°C, avec ensuite un refroidissement approximativement à température ambiante.

7. Procédé pour la préparation de la forme cristalline de la revendication 3, comprenant l'association d'une solution acétonique de N-(5-cyclopropyl-1-quinoléine-5-yl-1H-pyrazole-4-carbonyl)-guanidine et de N-méthylpyrrolidinone avec de l'acide méthanesulfonique à une température comprise dans l'intervalle d'environ 30°C à environ 60°C, avec ensuite un refroidissement à une température inférieure à environ 10°C.

8. Procédé pour la préparation de la forme cristalline de la revendication 3, comprenant l'association d'une solution acétonique de N-(5-cyclopropyl-1-quinoléine-5-yl-1H-pyrazole-4-carbonyl)-guanidine et de N-méthylpyrrolidinone avec de l'acide méthanesulfonique à une température comprise dans l'intervalle d'environ 30°C à environ 60°C, avec ensuite un refroidissement à une température d'environ 5°C.

9. Procédé pour la préparation de la forme cristalline de la revendication 4, comprenant la retrituration de la forme cristalline anhydre D de la revendication 3 dans de l'acétone à une température comprise dans l'intervalle d'environ 20°C à environ 25°C.

10. Procédé pour la préparation de la forme cristalline de la revendication 5, comprenant la retrituration de la forme cristalline anhydre A de la revendication 4 dans un mélange éthanol/eau ou isopropanol/eau à une température comprise dans l'intervalle d'environ 20°C à environ 25°C.

11. Procédé suivant la revendication 8, dans lequel un mélange éthanol/eau est utilisé et le rapport est un rapport d'environ 85 % à environ 95 % d'éthanol et d'environ 5 % à environ 15 % d'eau.

12. Hémihydrate de monomésylate de [5-cyclopropyl-1-(quinoléine-5-yl)-1H-pyrazole-4-carbonyl]guanidine.

13. Utilisation d'une forme cristalline suivant la revendication 1 pour la préparation d'un médicament destiné à réduire chez un mammifère l'altération tissulaire, résultant d'une ischémie ou hypoxie.

14. Utilisation suivant la revendication 13, dans laquelle le tissu est le tissu du coeur, du cerveau, du foie, des reins, des poumons, du tube digestif, des muscles squelettiques, de la rate, du pancréas, des nerfs, de la moelle épinière, de la rétine, du système vasculaire ou de l'intestin.

15. Utilisation suivant la revendication 14, dans laquelle la quantité de la forme cristalline de formule I est suffisante pour fournir une dose d'environ 0,01 mg/kg/jour à environ 50 mg/kg/jour.

16. Utilisation suivant la revendication 15, dans laquelle le mammifère est un être humain de sexe féminin ou masculin.

17. utilisation suivant la revendication 16, dans laquelle le médicament est destiné à être administré à titre prophylactique.

18. Utilisation suivant la revendication 16, dans laquelle le médicament est destiné à être administré avant une intervention chirurgicale.

19. Utilisation suivant la revendication 16, dans laquelle le médicament est destiné à être administré avant une intervention de chirurgie cardiaque.

20. Utilisation suivant la revendication 16, dans laquelle le médicament est destiné à être administré au cours d'une intervention chirurgicale.

21. Utilisation suivant la revendication 16, dans laquelle le médicament est destiné à être administré au cours d'une intervention de chirurgie cardiaque.

22. Utilisation suivant la revendication 16, dans laquelle le médicament est destiné à être administré dans les 24 heures suivant une intervention chirurgicale.

23. Utilisation suivant la revendication 16, dans laquelle l'altération tissulaire résultant d'une ischémie est une altération ischémique et est subie au cours d'une transplantation d'organe.

24. Utilisation suivant la revendication 16, dans laquelle le médicament est destiné à être administré pour prévenir une lésion ischémique myocardique périopératoire.

25. Composition pharmaceutique qui comprend une quantité thérapeutiquement efficace d'une forme cristalline de la revendication 1 et un support véhicule ou diluant pharmaceutiquement acceptable.

26. Composition pharmaceutique pour la réduction de l'altération tissulaire résultant d'une ischémie ou hypoxie, qui comprend une quantité thérapeutiquement efficace d'une forme cristalline de la revendication 1 et un support, véhicule ou diluant pharmaceutiquement acceptable.

27. Utilisation suivant la revendication 16, dans laquelle le médicament est destiné à être administré avant, pendant et après une intervention chirurgicale.

28. Utilisation suivant la revendication 16, dans laquelle le médicament est destiné à être administré avant, pendant et après une intervention de chirurgie cardiaque.

29. Forme cristalline suivant l'une quelconque des revendications 1 à 5, destinée à être utilisée comme agent pharmaceutique.
